# EUROPEAN PATENT APPLICATION

(11) **EP 3 698 629 A1**
(43) Date of publication of application: **26.08.2020**
(21) Application number: 20167865.3
(22) Date of filing: 09.06.2015
(51) Int. Cl.: A01N 1/02, A61B 5/15, B01L 3/00, C12M 1/00, C12N 5/00, C12N 5/078

(54) **STABILIZATION OF METABOLICALLY-ACTIVE CELLS IN A BLOOD SAMPLE AT AMBIENT TEMPERATURES**

(30) Priority: 10.06.2014 US 201462010237 P
(62) Divisional of application: 15807020.1
(71) Applicant: Biomatrica, Inc., San Diego, CA 92121 (US)
(72) Inventor: MULLER, Rolf, Del Mar, CA 92014 (US); RABAN, Robyn, San Diego, CA 92131 (US); WHITNEY, Scott, San Diego, CA 92126 (US); DIAZ, Paul, Riverside, CA 92505 (US)
(74) Representative: Glawe, Delfs, Moll

(57) **Abstract**

The present invention relates to the stabilization of one or more metabolically-active cell in a blood sample at ambient temperatures. In particular, formulations, compositions, articles of manufacture, kits and methods for substantially stable storage of one or more metabolically-active cell in a blood sample at ambient temperatures are provided.

## Description

### CROSS-REFERENCE

This application claims the benefit of U.S. Provisional Application No. 62/010,237, filed June 10, 2014, which is incorporated by reference herein in its entirety.

### BACKGROUND OF THE INVENTION

### 1. Technical Field

The present invention relates generally to stabilization of one or more metabolically-active cell in a blood sample at ambient temperatures. In particular, the invention relates to formulations, compositions, articles of manufacture, kits and methods for substantially stable storage of one or more metabolically-active cell in a blood sample at ambient temperatures.

### 2. Background

Whole blood is a complex mixture of cells, nucleic acids, proteins and various other analytes. In particular, blood components include, but are not limited to: cells, such as leukocytes (monocytes, lymphocytes and granulocytes), erythrocytes, thrombocytes and circulating tumor cells; nucleic acid molecules, such a circulating-free DNA (cfDNA); polypeptides, such as lipoproteins, albumin and serum proteins; and other various analytes.

Erythrocytes or red blood cells (RBCs) are flexible biconcave cells packed with hemoglobin which carry oxygen throughout the body. RBCs are the most dense whole blood component and thus, can be readily separated from whole blood, e.g., by centrifugation, or even by permitting settling under the force of gravity. Packed red cells can be transfused back into a donor patient (autologous transfusion) or transfused into a patient with a compatible blood type, e.g., having an appropriate A, B, AB, O and rh antigen type. Leukocytes or white blood cells (WBCs) are a diverse array of cell types, e.g., lymphocytes, macrophages, and polymorphonuclear neutrophils (PMNs), which are primarily involved with immune responses and fighting infections. WBCs are generally somewhat less dense than RBCs and, together with platelets, form a white "buffy coat" layer on top of RBCs during centrifugation of whole blood. Buffy coats can be a useful source of growth factors, blast cells and cytokines.

Compositions and methods for stabilizing, shipping and storing cells in a blood sample at ambient temperatures have been reported. These currently used compositions often fail to maintain the metabolic activity of the blood cells, and the stabilized cells do not retain a similar size and morphology as that found in whole blood, complicating subsequent analysis of these cells using automated cell separators and counters that sort and count cells based on predetermined cell size and shape.

Thus, there is a need to develop new formulations, compositions and methods for the stabilization of cells from blood that are maintained in an intact, metabolically-active state having a similar size and morphology to the cells in whole blood at ambient temperatures for prolonged periods. The formulations, compositions and methods of the present invention advantageously overcome the aforementioned limitations by providing intact, viable, metabolically active whole cells at ambient temperatures for a period of at least 18 days. In addition, the membrane-bound, cell surface proteins expressed on the substantially stored cells retain their native conformation in the blood sample facilitating analysis of these cells. These stabilized blood cells may be shipped and stored without the need for refrigeration or freezing, and are stable at ambient temperatures for extended periods of time, e.g., days, weeks, months or even years, facilitating the quantitation, analysis and/or use of various blood components for diagnostic and potential therapeutic applications.

### BRIEF SUMMARY OF THE INVENTION

Described herein, in some embodiments, are formulations for substantially stable storage of one or more metabolically-active cell in a blood sample at ambient temperatures, wherein the cell remains metabolically-active after storage at room temperature for a period of at least three days. In some embodiments, at least 80% of the stored cells remain metabolically-active after storage at room temperature for a period of at least three days. In some embodiments, the cells remain metabolically-active for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. In some embodiments, at least 80% of the cells remain metabolically-active at room temperature for a period of at least 18 days. In some embodiments, the formulation for substantially stable storage of metabolically-active cells in a blood sample at ambient temperatures comprises: a pH buffer; a chelating agent; and a peptide. In some embodiments, the pH buffer is selected from the group consisting of 2-(N-morpholino)ethanesulfonic acid (MES), 3-(N-morpholino)propanesulfonic acid (MOPS), 3-morpholino-2-hydroxypropanesulfonic acid (MOPSO), and a combination thereof. In some embodiments, the peptide is a di-peptide or a tri-peptide. In some embodiments, the di-peptide sequence is Ala-Gln or Gly-Gly. In some embodiments, the di-peptide sequence is Ala-Gln. In some embodiments, the di-peptide sequence is Gly-Gly. In some embodiments, the tri-peptide sequence is Gly-Gly-Gly. In some embodiments, the chelating agent is EDTA. In some embodiments, the one or more metabolically-active cell is selected from the group consisting of a leukocyte, an erythrocyte, a circulating tumor cell, and a combination thereof. In some embodiments, the formulation comprises: a pH buffer; a chelating agent; a phosphatase inhibitor; and a purine. In some embodiments, the phosphatase inhibitor is a serine-threonine phosphatase inhibitor. In some embodiments, the serine-threonine phosphatase inhibitor is 2-glycerol phosphate. In some embodiments, the purine is adenine or guanine. In some embodiments, the purine is adenine. In some embodiments, the purine is guanine. In some embodiments, the formulation further comprises a chelating agent, and at least one compound selected from the group consisting of a cationic compound, a zwitterionic compound, and a peptide. In some embodiments, the chelating agent is sodium gluconate. In some embodiments, the zwitterionic compound is a compound of formula (I): 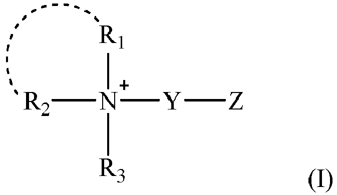 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. In some embodiments, the cationic compound is selected from the group consisting of:
(a) a compound of formula (II): 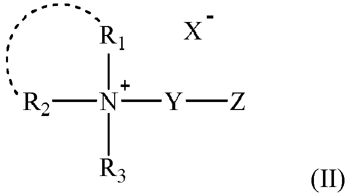 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 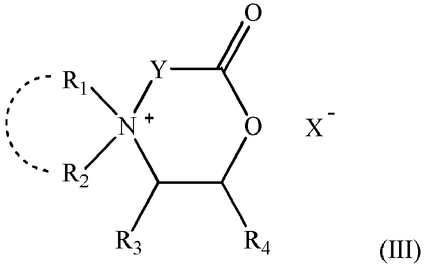 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 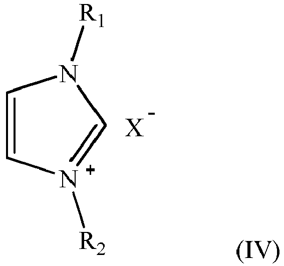 wherein R1 and R2 are independently selected from unsubstituted or substituted alkyl, unsubstituted or substituted aryl, unsubstituted or substituted arylalkyl; and X is a pharmaceutically acceptable anion. In some embodiments, R1 and R2 of a compound of formula (I), formula (II), or formula (III) form a morpholino ring, pyrrolidinium ring, a piperidinium ring, or an oxazinium ring. In some embodiments, the zwitterionic compound is selected from the zwitterionic compounds set forth in Table 1. In some embodiments, the cationic compound is selected from the cationic compounds set forth in Table 1. In some embodiments, the zwitterionic compound is a quaternary inner salt. In some embodiments, the quaternary salt is N,N-dimethyl-N-(2-hydroxyethyl)-3-ammonium-proprionate or N-ethyl-piperidinium-4-butylsulfonate. In some embodiments, the zwitterionic or cationic compound is selected from the group consisting of 4-(2-ethoxy-2-oxoethyl)-4-ethylmorpholin-4-ium bromide, N-(2-ethoxy-2-oxoethyl)-3-hydroxy-N,N-bis(2-hydroxyethyl)propan-1-aminium bromide, 2-ethoxy-N,N,N-triethyl-2-oxoethanaminium bromide, 2-((3-hydroxypropyl)dimethylammonio)acetate, 2-((2-hydroxypropyl)dimethylammonio) acetate, 2-(2-(hydroxymethyl)-1-methylpiperidinium-1-yl)acetate, 2-((2-hydroxyethyl)dimethylammonio)acetate, 2-((2,3-dihydroxypropyl) dimethylammonio)acetate, 1-(2-ethoxy-2-oxoethyl)-4-hydroxy-1-methylpiperidinium bromide, 2-(4-hydroxy-1-methylpiperidinium-1-yl)acetate, 2-ethoxy-N-(2-(2-hydroxyethoxy)ethyl)-N,N-dimethyl-2-oxoethanaminium bromide, 2-((2-(2-hydroxyethoxy)ethyl)dimethylammonio)acetate, 2-(bis(2-hydroxyethyl)-(methyl)ammonio)acetate & 4-(2-hydroxyethyl)-4-methyl-2-oxomorpholin-4-ium bromide, 2-(bis(2-hydroxyethyl)-(methyl)ammonio)acetate, 2-(4-(2-hydroxyethyl)morpholino-4-ium)acetate, 4-(2-ethoxy-2-oxoethyl)-4-methylmorpholin-4-ium bromide, 1-(2-ethoxy-2-oxoethyl)-1-methylpyrrolidinium bromide, 2-(benzyl(2-hydroxy-ethyl)(methyl)ammonio)acetate, 3-(2,3-dihydroxypropyl)-1-methyl-1H-imidazol-3-ium chloride, 1,3-dimethyl-1H-imidazol-3-ium methyl sulfate, N-benzyl-2-ethoxy-N,N-dimethyl-2-oxoethanaminium bromide, 1-(2-ethoxy-2-oxoethyl)-1-methylpiperidi-nium bromide, N-(2-ethoxy-2-oxoethyl)-N,N-dimethylbenzenaminium bromide, 1-(2-ethoxy-2-oxoethyl)-3-hydroxy-1-methylpiperidinium bromide, 3-(2-(2-hydroxyethoxy)ethyl)-1-methyl-1H imidazol-3-ium chloride, 3-(2-(2-(2-hydroxyethoxy)ethoxy)ethyl)-1-methyl-1H-imidazol-3-ium chloride, 1-methyl-3-tetradecyl-1H-imidazol-3-ium bromide, N-(2-ethoxy-2-oxoethyl)-N,N-dimethylcyclo-hexanaminium bromide, and 3-((2-hydroxy-ethyl)dimethyl-ammonio)pro-panoate. In some embodiments, the peptide has the amino acid sequence of Ala-Gin, Gly-Gly or Gly-Gly-Gly. In some embodiments, the peptide has the amino acid sequence of Ala-Gln. In some embodiments, the peptide has the amino acid sequence of Gly-Gly. In some embodiments, the peptide has the amino acid sequence of Gly-Gly-Gly. In some embodiments, the formulation further comprises an acetate buffer, sucralose, glucose, potassium chloride, myo-inositol, N-methylglucamine, magnesium chloride, or a combination thereof. In some embodiments, the one or more metabolically-active cell is selected from the group consisting of a leukocyte, an erythrocyte, a circulating tumor cell, and a combination thereof. In some embodiments, the one or more metabolically-active cell is a leukocyte. In some embodiments, the one or more metabolically-active cell is an erythrocyte. In some embodiments, the one or more metabolically-active cell is a circulating tumor cell. In some embodiments, the formulation is selected from the formulations set forth in Table 2. In certain embodiments, the pH buffer is selected from the group consisting of 2-(N-morpholino)ethanesulfonic acid (MES), 3-(N-morpholino)propanesulfonic acid (MOPS), and 3-morpholino-2-hydroxypropanesulfonic acid (MOPSO), the chelating agent is EDTA, and the peptide is a di-peptide or a tri-peptide. In certain embodiments, the phosphatase inhibitor is 2-glycerolol phosphate and the purine is adenine or guanine. In certain embodiments, the phosphatase inhibitor is 2-glycerol phosphate and the purine is adenine.

Described herein, in some embodiments, are compositions of a substantially, stably stored one or more metabolically-active leukocyte, comprising one or more metabolically-active leukocyte admixed with a formulation for substantially stable storage of one or more metabolically-active cell. In certain embodiments, the one or more metabolically-active leukocyte is purified and stored in the formulations for substantially stable storage.

Described herein, in some embodiments, are compositions of a substantially, stably stored one or more metabolically-active erythrocyte comprising one or more metabolically-active erythrocyte admixed with a formulation for substantially stable storage of one or more metabolically-active cell. In certain embodiments, the one or more metabolically-active erythrocyte is purified and stored in the formulations for substantially stable storage.

Described herein, in some embodiments, are compositions of a substantially, stably stored one or more metabolically-active circulating tumor cell comprising on or more metabolically-active circulating tumor cell admixed with a formulation for substantially stable storage of one or more metabolically-active cell.In certain embodiments, the one or more metabolically-active circulating tumor cell is purified and stored in the formulations for substantially stable storage.

Described herein, in some embodiments, are articles of manufacture comprising a formulation of any one of the formulations described herein contained within a blood collection tube. In some embodiments, the blood collection tube is an evacuated blood collection tube.

Described herein, in some embodiments, are kits comprising one of the articles of manufacture and a package insert.

Described herein, in some embodiments, are methods for substantially stable storage of one or more metabolically-active cell in a blood sample at ambient temperatures comprising: admixing a sample of collected blood from a subject with for substantially stable storage of one or more metabolically-active cell in a blood sample at ambient temperatures as provided herein, wherein the one or more cell remain metabolically-active at room temperature for a period of at least three days. In some embodiments, at least 80% of the cells remain metabolically-active at room temperature for a period of at least three days. In other embodiments of the method, the cells remain metabolically-active for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days. In some embodiments, the cell remains metabolically-active at room temperature for a period of at least 18 days. In some embodiments, the cell is a leukocyte, an erythrocyte, a circulating tumor cell, or a combination thereof. In some embodiments, the subject is an animal. In some embodiments, the subject is a mammal. In some embodiments, the subject is a human. In certain embodiments, the blood sample is admixed with the stabilization formulation at the time the blood is collected from the subject to substantially stabilize the one or more metabolically-active cell.

### BRIEF DESCRIPTION OF THE FIGURES

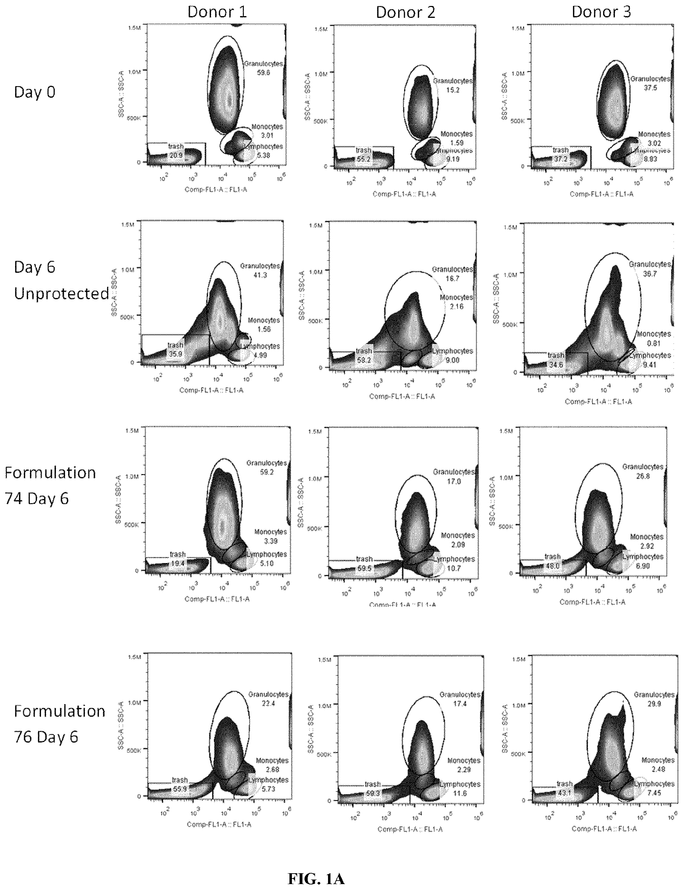

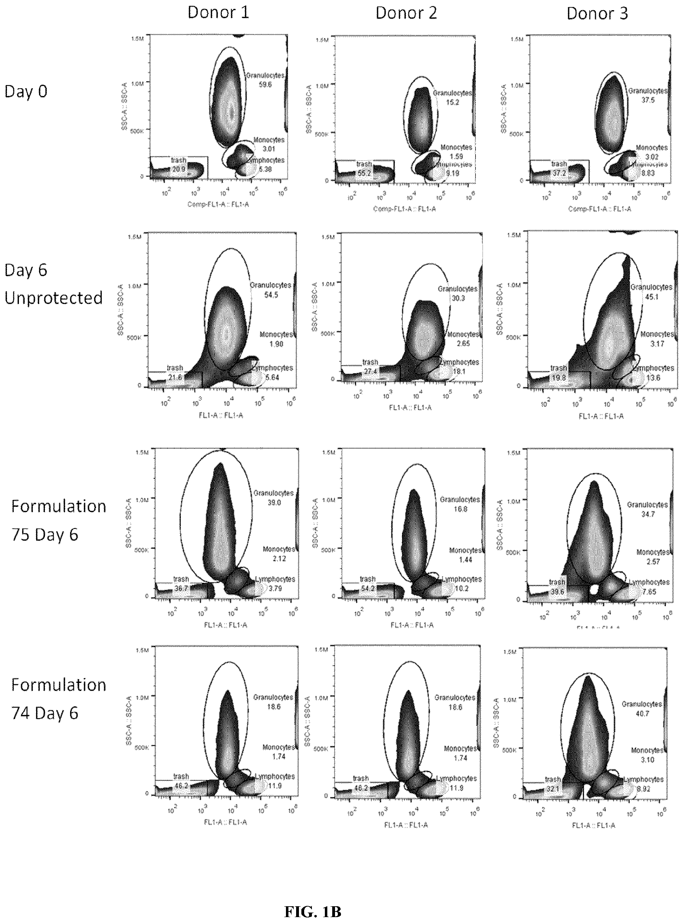

Figs. 1A and 1B show FACS scatter plots of a 3 donor study demonstrating that formulations and compositions of the present invention are capable of stabilizing CD45 positive leukocytes in a blood sample at ambient temperatures or at 4°C after storage for 6 days. Fig. 1A depicts stabilization at ambient temperatures with exemplary formulations versus an unprotected sample, and Fig. 1B depicts stabilization at 4°C with exemplary formulations versus an unprotected sample.

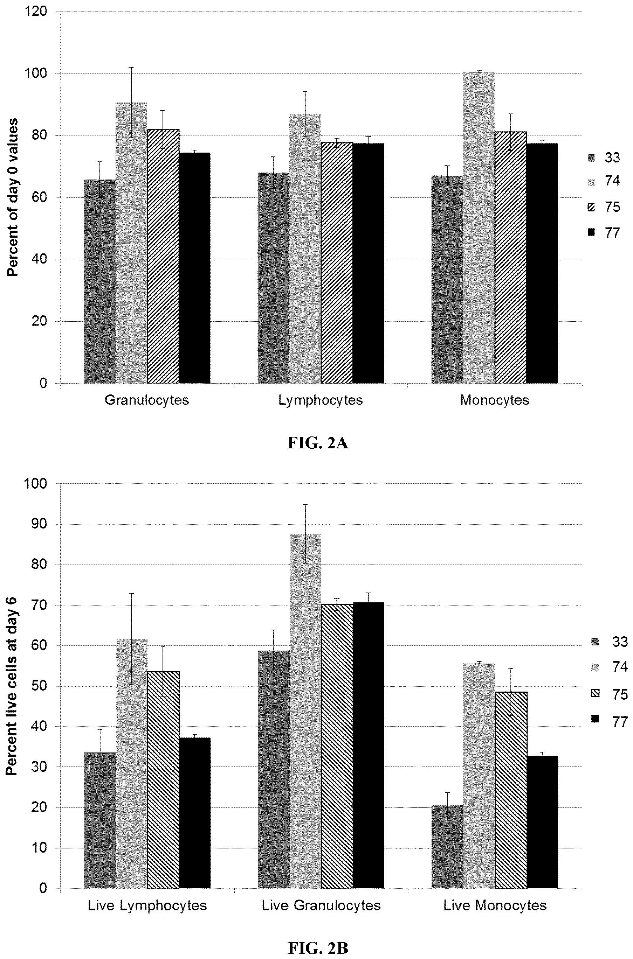

Figs. 2A and 2B provide bar graphs demonstrating that formulations and compositions of the present invention are capable of stabilizing CD45-positive total leukocyte and viable leukocyte populations comprising of granulocytes, lymphocytes and monocytes in a blood sample after storage for 6 days at 4°C versus an unprotected sample.

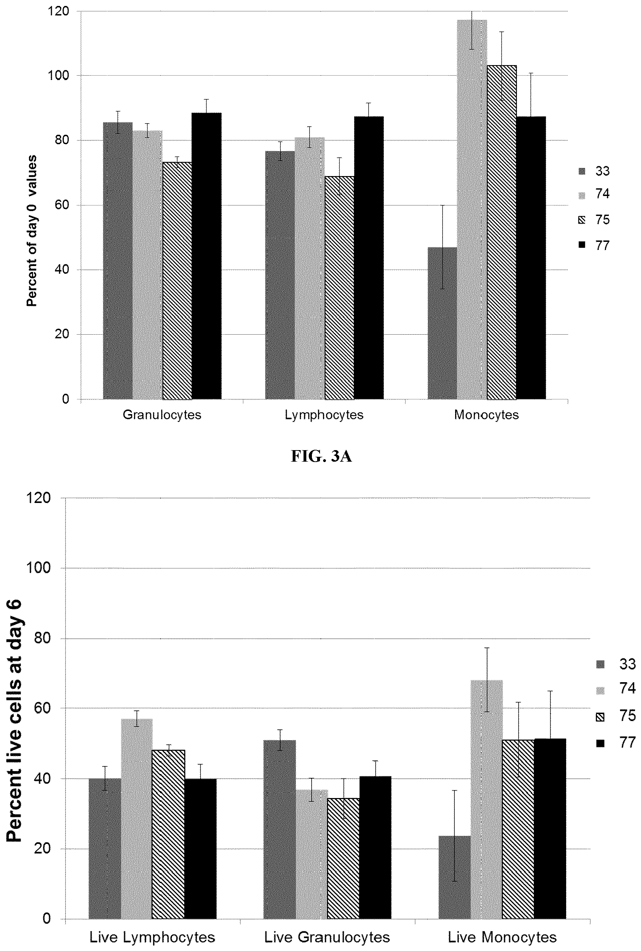

Figs. 3A and 3B provide bar graphs demonstrating that formulations and compositions of the present invention are capable of stabilizing CD45-positive total leukocyte and viable leukocyte populations comprising of granulocytes, lymphocytes and monocytes in a blood sample after storage for 6 days at room temperature versus an unprotected sample.

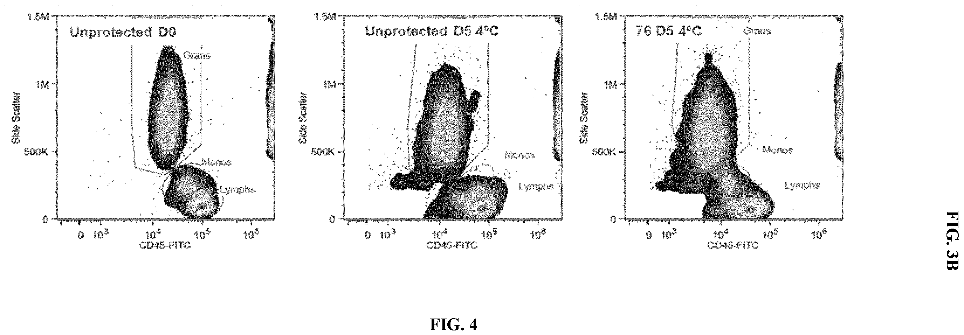

Fig. 4 shows a FACS scatter plot demonstrating that formulations and compositions of the present invention are capable of stabilizing monocyte populations in a blood sample after storage for 5 days at 4°C versus an unprotected sample.

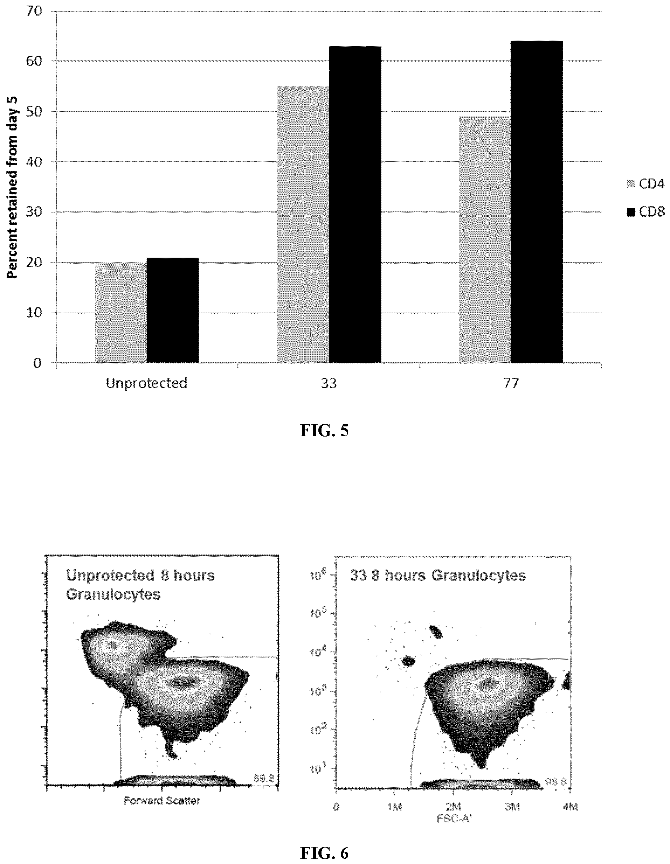

Fig. 5 shows a bar graph demonstrating that formulations and compositions of the present invention are capable of stabilizing CD4- and CD8-positive T cells/lymphocytes in a blood sample after storage for 5 days at room temperature versus an unprotected sample.
Fig. 6 shows a FACS scatter plot demonstrating that formulations and compositions of the present invention are capable of stabilizing viable CD66b-positive granulocyte populations in a blood sample after storage for 8 hours at room temperature versus an unprotected sample.

### DETAILED DESCRIPTION OF THE INVENTION

According to the present invention, there are provided formulations, compositions, articles of manufacture, kits and methods for substantially stable storage of one or more metabolically-active cell in a blood sample at ambient temperatures. In one aspect, the formulations described herein beneficially maintain the integrity of intact, metabolically active, viable blood cells of similar size and shape as found in whole blood, e.g., leukocytes, erythrocytes, and circulating tumor cells. These viable cells may advantageously be analyzed by flow cytometry or fluorescence activated cell sorting (FACS) analysis using antibodies generated against a native wild type membrane proteins, receptors and cell surface proteins not possible using other storage formulations that denature these cell surface proteins.

Unless defined otherwise, all technical and scientific terms used herein have the same meaning as is commonly understood by one of skill in the art to which this invention belongs. All patents, patent applications and publications referred to herein are incorporated by reference in their entirety.

As used in this specification and the appended claims, the singular forms "a", "an", and "the" include plural references unless the context clearly dictates otherwise. Thus, for example, references to "the method" includes one or more methods, and/or steps of the type described herein which will become apparent to those persons skilled in the art upon reading this disclosure and so forth.

"About" as used herein when referring to a measurable value such as an amount, a temporal duration, and the like, is meant to encompass variations of ±20% or ±10%, or ±5%, or even ±1% from the specified value, as such variations are appropriate for the disclosed compositions or to perform the disclosed methods.

### FORMULATIONS AND COMPOSITIONS FOR STABILIZING ONE OR MORE METABOLICALLY-ACTIVE CELL IN A BLOOD SAMPLE AT AMBIENT TEMPERATURES

In one aspect of the present invention, formulations are provided for substantially stable storage of one or more metabolically-active cell, e.g., a leukocyte, an erythrocyte, and/or a circulating tumor cell in a blood sample at ambient temperatures. In certain embodiments, the cell remains metabolically-active at room temperature for a period of at least three days. In other embodiments, the cells remain metabolically-active for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

The term "ambient temperature" as used herein refers to common indoor room temperatures. In some embodiments, ambient temperature is 15 to 32°C. In some embodiments, ambient temperature is 20 to 27°C.

In certain other embodiments, the formulations for substantially stable storage of one or more metabolically-active cell in a blood sample comprises: a pH buffer; a chelating agent; and a peptide. In some embodiments, the peptide is a di- or tri-peptide. In some embodiments, the di-peptide is alanine-glutamine. In some embodiments, the di-peptide is glycine-glycine. In some embodiments, the tri-peptide is glycine-glycine-glycine. Other formulations comprise: a pH buffer; a phosphatase inhibitor; and a purine. In some embodiments, the purine is adenine or guanine. In some embodiments, these formulations further comprise a chelating agent and at least one of a cationic compound, zwitterionic compound or a peptide. In some embodiments, the formulation further comprises an acetate buffer, sucralose, glucose, potassium chloride, myo-inositol, N-methylglucamine, magnesium chloride, or a combination thereof.

In another aspect, compositions are provided comprising a blood sample admixed with a stabilization formulation as provided herein to produce substantially stable leukocytes, erythrocytes, or circulating tumor cells in a whole blood preparation. In other aspects, a composition comprising isolated or purified leukocytes, erythrocytes, or circulating tumor cells admixed with the stabilization formulation are provided.

### FORMULATION REAGENTS

### A. pH Buffers

According to certain embodiments, the herein described formulations and compositions for substantially stable storage of one or more cell in a blood sample include one or more pH buffers. In some embodiments, the pH buffer is any of a large number of compounds known in the art for their ability to resist changes in the pH of a solution, such as an aqueous solution, in which the pH buffer is present. Selection of one or more particular pH buffers for inclusion in a stable storage composition may be done based on the present disclosure and according to routine practices in the art, and may be influenced by a variety of factors including the pH that is desirably to be maintained, the nature of the sample to be stabilized, the solvent conditions to be employed, the other components of the formulation to be used, and other criteria. For example, typically a pH buffer is employed at a pH that is within about 0.1, 0.2, 0.3, 0.4, 0.5, 0.6, 0.7, 0.8, 0.9 or 1.0 pH unit of a proton dissociation constant (pKa) that is a characteristic of the buffer.

Non-limiting examples of pH buffers include citric acid, tartaric acid, malic acid, sulfosalicylic acid, sulfoisophthalic acid, oxalic acid, borate, CAPS (3-(cyclohexylamino)-1-propanesulfonic acid), CAPSO (3-(cyclohexylamino)-2-hydroxy-1-propanesulfonic acid), EPPS (4-(2-hydroxyethyl)-1-piperazinepropanesulfonic acid), HEPES (4-(2-hydroxyethyl)piperazine-1-ethanesulfonic acid), MES (2-(N-morpholino)ethanesulfonic acid), MOPS (3-(N-morpholino)propanesulfonic acid), MOPSO (3-morpholino-2-hydroxypropanesulfonic acid), PIPES (1,4-piperazinediethanesulfonic acid), TAPS (N-[tris(hydroxymethyl)methyl]-3-aminopropanesulfonic acid), TAPSO (2-hydroxy-3-[tris(hydroxymethyl)methylamino]-1-propanesulfonic acid), TES (N-[tris(hydroxymethyl)methyl]-2-aminoethanesulfonic acid), bicine (N,N-bis(2-hydroxyethyl)glycine), tricine (N-[tris(hydroxymethyl)methyl]glycine), tris (tris(hydroxymethyl)aminomethane) and bis-tris (2-[bis(2-hydroxyethyl)amino]-2-(hydroxymethyl)-1,3-propanediol). Certain embodiments contemplated herein, including a number of those set forth in Table 2, feature a formulation having a pH of about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0.

### B. Cationic Compounds and Zwitterionic Compounds

In some embodiments, the formulation contains a cationic compound, a zwitterionic compound, or a combination thereof. In certain embodiments, the zwitterionic compound is a quaternary inner salt. In certain embodiments, the formulation for substantially stable storage of one or more cell in a blood sample at ambient temperatures, including those set forth in Table 2, the quaternary inner salt is N,N-dimethyl-N-(2-hydroxyethyl)-3-ammonium-proprionate or N-ethyl-piperidinium-4-butylsulfonate. In some embodiments, the quaternary salt is N,N-dimethyl-N-(2-hydroxyethyl)-3-ammonium-proprionate or N-ethyl-piperidinium-4-butylsulfonate, and is present at concentrations of about 1.0 - 100 mg/mL, or 1.0 - 50 mg/mL, or about 10.0 - 50 mg/ml. In some embodiments, the quaternary inner salt is one or more of those disclosed in WO 2012/018638.

In some embodiments, the zwitterionic compound is a compound of formula (I): 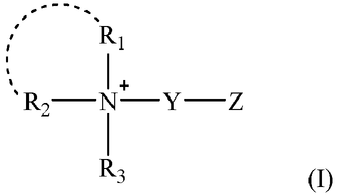 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-. In some embodiments, R1 and R2 form a morpholino ring, pyrrolidinium ring, a piperidinium ring, or an oxazinium ring.

In some embodiments, the cationic compound is selected from the group consisting of:
(a) a compound of formula (II): 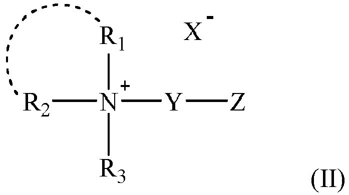 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
(b) a compound of formula (III): 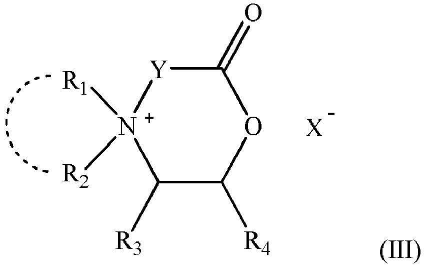 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
(c) a compound of formula (IV): 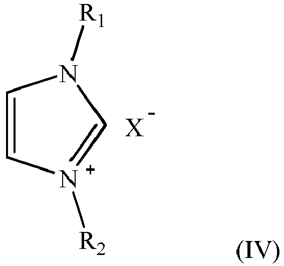 wherein R1 and R2 are independently selected from unsubstituted or substituted alkyl, unsubstituted or substituted aryl, unsubstituted or substituted arylalkyl; and X is a pharmaceutically acceptable anion. In some embodiments, R1 and R2 of a compound of formula (II) or formula (III) form a morpholino ring, pyrrolidinium ring, a piperidinium ring, or an oxazinium ring.

An "alkyl" group refers to an aliphatic hydrocarbon group. The alkyl moiety includes a "saturated alkyl" group, which means that it does not contain any alkene or alkyne moieties. The alkyl moiety also includes an "unsaturated alkyl" moiety, which means that it contains at least one alkene or alkyne moiety. An "alkene" moiety refers to a group that has at least one carbon-carbon double bond, and an "alkyne" moiety refers to a group that has at least one carbon-carbon triple bond. The alkyl moiety, whether saturated or unsaturated, includes branched, straight chain, or cyclic moieties. Depending on the structure, an alkyl group includes a monoradical or a diradical (i.e., an alkylene group), and if a "lower alkyl" having 1 to 6 carbon atoms. As used herein, C1-Cx includes C1-C2, C1-C3 ... C1-Cx. The "alkyl" moiety optionally has 1 to 10 carbon atoms (whenever it appears herein, a numerical range such as "1 to 10" refers to each integer in the given range; e.g., "1 to 10 carbon atoms" means that the alkyl group is selected from a moiety having 1 carbon atom, 2 carbon atoms, 3 carbon atoms, etc., up to and including 10 carbon atoms, although the present definition also covers the occurrence of the term "alkyl" where no numerical range is designated). The alkyl group of the compounds described herein may be designated as "C1-C4 alkyl" or similar designations. By way of example only, "C1-C4 alkyl" indicates that there are one to four carbon atoms in the alkyl chain, i.e., the alkyl chain is selected from among methyl, ethyl, propyl, iso-propyl, n-butyl, iso-butyl, sec-butyl, and t-butyl. Thus C1-C4 alkyl includes C1-C2 alkyl and C1-C3 alkyl. Alkyl groups are optionally substituted or unsubstituted. Typical alkyl groups include, but are in no way limited to, methyl, ethyl, propyl, isopropyl, butyl, isobutyl, tertiary butyl, pentyl, hexyl, ethenyl, propenyl, butenyl, cyclopropyl, cyclobutyl, cyclopentyl, cyclohexyl, and the like.

As used herein, the term "ring" refers to any covalently closed structure. Rings include, for example, carbocycles (e.g., aryls and cycloalkyls), heterocycles (e.g., heteroaryls and non-aromatic heterocycles), aromatics (e.g. aryls and heteroaryls), and non-aromatics (e.g., cycloalkyls and non-aromatic heterocycles). Rings can be optionally substituted. Rings can be monocyclic or polycyclic.

The term "aryl" used alone or as part of a larger moiety as in "arylalkyl", "arylalkoxy", or "aryloxyalkyl", refers to a monocyclic, bicyclic or tricyclic, carbon ring system, that includes fused rings, wherein at least one ring in the system is aromatic. The term "aryl" may be used interchangeably with the term "aryl ring". In one embodiment, aryl includes groups having 6-12 carbon atoms. In another embodiment, aryl includes groups having 6-10 carbon atoms. Examples of aryl groups include phenyl, naphthyl, anthracyl, phenanthrenyl, naphthacenyl, 1,2,3,4-tetrahydronaphthalenyl, 1H-indenyl, 2,3-dihydro-1H-indenyl, and the like. A particular aryl is phenyl. In another embodiment aryl includes indanyl, naphthyl, and tetrahydronaphthyl, and the like, where the radical or point of attachment is on an aromatic ring.

The term "optionally substituted" or "substituted" means that the referenced group may be substituted with one or more additional group(s) individually and independently selected from alkyl, cycloalkyl, aryl, heteroaryl, heteroalicyclic, hydroxy, alkoxy, aryloxy, alkylthio, arylthio, alkylsulfoxide, arylsulfoxide, alkylsulfone, arylsulfone, cyano, halo, acyl, nitro, haloalkyl, fluoroalkyl, amino, including mono- and di-substituted amino groups, and the protected derivatives thereof. By way of example an optional substituents may be LsRs, wherein each Ls is independently selected from a bond, -O-, -C(=O)-, -S-, -S(=O)-, -S(=O)₂-, -NH-, -NHC(O)-, - C(O)NH-, S(=O)₂NH-, -NHS(=O)₂, -OC(O)NH-, -NHC(O)O-, -(substituted or unsubstituted C1-C6 alkyl), or -(substituted or unsubstituted C2-C6 alkenyl); and each Rs is independently selected from H, (substituted or unsubstituted C1-C4 alkyl), (substituted or unsubstituted C3-C6 cycloalkyl), heteroaryl, or heteroalkyl.

In embodiments of the formulations described herein, including those set forth in Table 2, the cationic compound is selected from one of the exemplary cationic compounds of Table 1. In embodiments of the formulations described herein, including those set forth in Table 2, the zwitterionic compound is selected from one of the exemplary zwitterionic compounds of Table 1.

**TABLE 1. Exemplary Cationic and Zwitterionic Compounds for Stabilizing Cells in a Blood Sample at Ambient Temperatures**

| | | |
|---|---|---|
| D201 | 4-(2-ethoxy-2-oxoethyl)-4-ethylmorpholin-4-ium bromide | Cationic Compound |
| D202 | N-(2-ethoxy-2-oxoethyl)-3-hydroxy-N,N-bis(2-hydroxyethyl)propan-1-aminium bromide | Cationic Compound |
| D203 | 2-ethoxy-N,N,N-triethyl-2-oxoethanaminium bromide | Cationic Compound |
| D204 | 2-((3-hydroxypropyl)dimethylammonio)acetate | Zwitterion |
| D205 | 2-((2-hydroxypropyl)dimethylammonio)acetate | Zwitterion |
| D206 | 2-(2-(hydroxymethyl)-1-methylpiperidinium-1-yl)acetate | Zwitterion |
| D207 | 2-((2-hydroxyethyl)dimethylammonio)acetate | Zwitterion |
| D208 | 2-((2,3-dihydroxypropyl) dimethylammonio)acetate | Zwitterion |
| D209 | 1-(2-ethoxy-2-oxoethyl)-4-hydroxy-1-methylpiperidinium bromide | Cationic Compound |
| D210 | 2-(4-hydroxy-1-methylpiperidinium-1-yl)acetate | Zwitterion |
| D211 | 2-ethoxy-N-(2-(2-hydroxyethoxy)ethyl)-N,N-dimethyl-2-oxoethanaminium bromide | Cationic Compound |
| D212 | 2-((2-(2-hydroxyethoxy)ethyl)dimethylammonio)acetate | Zwitterion |
| D213 | 4-(2-hydroxyethyl)-4-methyl-2-oxomorpholin-4-ium bromide | Cationic Compound |
| D214 | 2-(bis(2-hydroxyethyl)-(methyl)ammonio)acetate | Zwitterion |
| D215 | 2-(4-(2-hydroxyethyl)morpholino-4-ium)acetate | Zwitterion |
| D216 | 2-(4-(2-hydroxyethyl)morpholino-4-ium)acetate | Zwitterion |
| D217 | 4-(2-ethoxy-2-oxoethyl)-4-methylmorpholin-4-ium bromide | Cationic Compound |
| D218 | 1-(2-ethoxy-2-oxoethyl)-1-methylpyrrolidinium bromide | Cationic Compound |
| D219 | 2-(benzyl(2-hydroxy-ethyl)(methyl)ammonio)acetate | Zwitterion |
| D220 | 3-(2,3-dihydroxypropyl)-1-methyl-1H-imidazol-3-ium chloride | Cationic Compound |
| D221 | 1,3-dimethyl-1H-imidazol-3-ium methyl sulfate | Cationic Compound |
| D222 | 3-(2-ethoxy-2-oxoethyl)-1-methyl-1H-imidazol-3-ium bromide | Cationic Compound |
| D223 | 2-(1-(2-hydroxyethyl) pyrrolidinium-1-yl) acetate | Zwitterion |
| D224 | N-benzyl-2-ethoxy-N,N-dimethyl-2-oxoethanaminium bromide | Cationic Compound |
| D225 | 2-ethoxy-N,N-diethyl-N-methyl-2-oxoethanaminium bromide | Cationic Compound |
| D226 | N-(2-ethoxy-2-oxoethyl)-N,N-dimethylbutan-1-aminium bromide | Cationic Compound |
| D227 | 1-(2-ethoxy-2-oxoethyl)-1-methylpiperidinium bromide | Cationic Compound |
| D228 | N-(2-ethoxy-2-oxoethyl)-N,N-dimethylbenzenaminium bromide | Cationic Compound |
| D229 | 1-(2-ethoxy-2-oxoethyl)-3-hydroxy-1-methylpiperidinium bromide | Cationic Compound |
| D230 | 3-(2-(2-hydroxyethoxy)ethyl)-1-methyl-1H-imidazol-3-ium chloride | Cationic Compound |
| D231 | 3-(2-(2-(2-hydroxyethoxy)ethoxy)ethyl)-1-methyl-1H-imidazol-3-ium chloride | Cationic Compound |
| D232 | 1-methyl-3-tetradecyl-1H-imidazol-3-ium bromide | Cationic Compound |
| D233 | N-(2-ethoxy-2-oxoethyl)-N,N-dimethylcyclohexanaminium bromide | Cationic Compound |
| D234 | 3-((2-hydroxy-ethyl)dimethyl-ammonio)propanoate | Zwitterion |

### C. Peptides

The herein described formulations for substantially stable storage of one or more metabolically-active cell in a blood sample at ambient temperatures contain a peptide, in certain embodiments. Advantageously, it has been determined that formulations, including those set forth in Table 2, comprising a stabilizing amount of a small di- or tri-peptide, e.g., 50 mM or 200 mM, in the presence of a pH buffer are unexpectedly capable of substantially stabilizingmetabolically-active cells in a blood sample at ambient temperatures and for period of time that exceed cold storage of these cells. In some embodiments, the di-peptide has the amino acid sequence aa-aa, wherein aa is any of the 20 natural or any unnatural amino acids. In some embodiments, the di-peptide is alanine-glutamine. In some embodiments, the di-peptide is glycine-glycine. In some embodiments, the tri-peptide has the amino acid sequence aa-aa-aa, wherein aa is any of the 20 natural or any unnatural amino acids. In some embodiments, the tri-peptide is glycine-glycine-glycine.

### D. Phosphatase Inhibitors

The herein described formulations for substantial stable storage one or more metabolically-active cell in a blood sample at ambient temperatures may, in certain embodiments, contain a phosphatase inhibitor. In certain embodiments, the phosphatase inhibitor is an inhibitor of the serine -threonine class of phosphatases. In some embodiments, the phosphatase inhibitor is 2-glycerol phosphate. In some embodiments, the phosphatase inhibitor is present at a concentration of about 1.0 - 100 mM, or about 25 - 75 mM, or about about 50 mM. In some embodiments, the phosphatase inhibitor is 2-glycerol phosphate, and is present at a concentration of about 1.0 - 100 mM, or about 25 - 75 mM, or about about 50 mM

### E. Chelating Agents

Chelating agents or chelators are, according to certain embodiments, included in the presently described composition for substantially stable storage of viable, intact cells in a blood sample. Such chelating agents are known to those familiar with the art for their ability to complex with and hinder the reactivity of metal cations. Exemplary chelating agents include diethylenetriaminepentaacetic acid (DTPA), ethylenediaminetetraacetic acid (EDTA), ethylene glycol tetraacetic acid (EGTA), trans-1,2-diaminocyclohexane-N,N,N',N'-tetraacetic acid (CDTA), 1,2-bis(2-aminophenoxy)ethane-N,N,N',N'-tetraacetic acid (BAPTA), 1,4,7,10-tetraazacyclododecane-1,4,7,10-tetraacetic acid (DOTA), N-(2-hydroxyethyl)ethylenediamine-N,N',N'-triacetic acid, sodium gluconate, and nitrilotriacetic acid (NTA). In some embodiments, the chelating agent is sodium gluconate and is present at a concentration of about 1.0 - 50 mM, or about 10 - 40 mM, or about 25 mM.

### F. Purines

In some embodiments, a purine is included in the presently described composition for substantially stable storage of viable, intact cells in a blood sample. In some embodiments, the purine is adenine, guanine, or both. In some embodiments, the purine is adenine. In some embodiments, the purine is guanine.

### EXEMPLARY FORMULATIONS FOR STABILIZATION OF ONE OR MORE METABOLICALLY-ACTIVE CELL IN A BLOOD SAMPLE AT AMBIENT TEMPERATURES

In certain embodiments, formulations are provided for substantially stable storage of one or more metabolically-active cell in a blood sample at ambient temperatures. In certain embodiments, the formulation for substantially stable storage of one or more metabolically-active cell in a blood sample comprises: a pH buffer; a chelating agent; and a peptide. In certain embodiments, the formulation for substantially stable storage of one or more metabolically-active cell in a blood sample comprises: a pH buffer; a chelating agent; and a di- or tri-peptide. In certain embodiments, the formulation for substantially stable storage of one or more metabolically-active cell in a blood sample comprises: a pH buffer; a chelating agent; and an alanine-glutamine di-peptide, a glycine-glycine di-peptide or a glycine-glycine-glycine tri-peptide. In certain embodiments, the formulation for substantially stable storage of one or more metabolically-active cell in a blood sample comprises: a pH buffer; a phosphatase inhibitor; and a purine. In certain embodiments, the phosphatase inhibitor is a serine-threonine phosphatase inhibitor, the purine is adenine or guanine. In certain embodiments, the phosphatase inhibitor is 2-glycerol phosphate and the purine is adenine. In other embodiments, the formulations further comprise: a chelating agent; and at least one of a cationic compound, a zwitterionic compound, or a peptide. In other embodiments, the formulations further comprise a sodium gluconate and at least one of a cationic compound, zwitterionic compound or a peptide. In some embodiments, the formulation further comprises an acetate buffer, sucralose, glucose, potassium chloride, myo-inositol, N-methylglucamine, magnesium chloride, or a combination thereof. In some embodiments, the zwitterionic compound is a quaternary inner salt, and the peptides are di- or tri-peptides having the amino acid sequence Ala-Gln, Gly-Gly, or Gly-Gly-Gly, respectively.

Exemplary formulations for substantially stable storage of one or more metabolically-active cell in a blood sample at ambient temperatures are shown in Table 2.

**TABLE 2**

| Exemplary Formulations for Stabilizing One or More Metabolically-active Cell in a Blood Sample at Ambient Temperatures | | | | |
|---|---|---|---|---|
| **Formulation No.** | **Composition** | | **Formulation No.** | **Composition** |
| 1 | 200 mM Gly-Gly-Gly, 4.4 mM K2-EDTA, 50 mM MES, pH 6.6 | | 40 | 50 mM MOPS, 50 mM 2-Glycerol Phosphate disodium salt, 50 mM D-216, 4.4 mM K2-EDTA, 2 mM Adenine, pH 7.4 |
| 2 | 200 mM Gly-Gly, 4.4 mM K2-EDTA, 50 mM MOPS, pH 6.9 | | 41 | 50 mM MOPS, 50 mM 2-Glycerol Phosphate disodium salt, 50 mM D-217, 4.4 mM K2-EDTA, 2 mM Adenine, pH 7.4 |
| 3 | 200 mM Ala-Gln, 4.4 mM K2-EDTA, 50 mM MES, pH 6.6 | | 42 | 50 mM MOPS, 50 mM 2-Glycerol Phosphate disodium salt, 50 mM D-218, 4.4 mM K2-EDTA, 2 mM Adenine, pH 7.4 |
| 4 | 200 mM Ala-Gln, 4.4 mM K2-EDTA, 50 mM MOPS, pH 6.9 | | 43 | 50 mM MOPS, 50 mM 2-Glycerol Phosphate disodium salt, 50 mM D-219, 4.4 mM K2-EDTA, 2 mM Adenine, pH 7.4 |
| 5 | 50 mM MOPS, 50 mM 2-Glycerol Phosphate disodium Salt, 4.4 mM K2-EDTA, 100 mM D-201 | | 44 | 50 mM MOPS, 50 mM 2-Glycerol Phosphate disodium salt, 50 mM D-220, 4.4 mM K2-EDTA, 2 mM Adenine, pH 7.4 |
| 6 | 50 mM MOPS, 50 mM 2-Glycerol Phosphate disodium Salt, 4.4 mM K2-EDTA, 100 mM D-202 | | 45 | 50 mM MOPS, 50 mM 2-Glycerol Phosphate disodium salt, 100 mM Ala-Gln, 4.4 mM K2-EDTA, 2 mM Adenine, 25 mM NaOAc, 5 mM KCl, 25 mM Sodium Gluconate, 3 mM MgCl₂ pH 7.4 |
| 7 | 50 mM MOPS, 50 mM 2-Glycerol Phosphate disodium Salt, 4.4 mM K2-EDTA, 100 mM D-203 | | 46 | 50 mM MOPS, 50 mM 2-Glycerol Phosphate disodium salt, 50 mM Ala-Gln, 4.4 mM K2-EDTA, 2 mM Adenine, 25 mM NaOAc, 5 mM KCl, 25 mM Sodium Gluconate, 3 mM MgCl₂ pH 7.4 |
| 8 | 50 mM MOPS, 50 mM 2-Glycerol Phosphate disodium Salt, 4.4 mM K2-EDTA, 100 mM D-204 | | 47 | 50 mM MOPS, 50 mM 2-Glycerol Phosphate disodium salt, 65 mM Ala-Gln, 4.4 mM K2-EDTA, 2 mM Adenine, 25 mM NaOAc, 5 mM KCl, 25 mM Sodium Gluconate, 3 mM MgCl₂ pH 7.4 |
| 9 | 50 mM MOPS, 50 mM 2-Glycerol Phosphate disodium Salt, 4.4 mM K2-EDTA, 100 mM D-205 | | 48 | 25 mM MOPS, 25 mM 2-Glycerol Phosphate disodium salt, 65 mM Ala-Gln, 4.4 mM K2-EDTA, 2 mM Adenine, 25 mM NaOAc, 5 mM KCl, 25 mM Sodium Gluconate, 3 mM MgCl₂, pH 7.4 |
| 10 | 50 mM MOPS, 50 mM 2-Glycerol Phosphate disodium Salt, 4.4 mM K2-EDTA, 100 mM D-206 | | 49 | 50 mM MOPS, 25 mM 2-Glycerol Phosphate disodium salt, 25 mM D-221, 4.4 mM K2-EDTA, 2 mM Adenine, 25 mM NaOAc, 5 mM KCl, 25 mM Sodium Gluconate, 3 mM MgCl₂, pH 7.4 |
| 11 | 50 mM MOPS, 50 mM 2-Glycerol Phosphate disodium Salt, 4.4 mM K2-EDTA, 100 mM D-207 | | 50 | 50 mM MOPS, 25 mM 2-Glycerol Phosphate disodium salt, 25 mM D-222, 4.4 mM K2-EDTA, 2 mM Adenine, 25 mM NaOAc, 5 mM KCl, 25 mM Sodium Gluconate, 3 mM MgCl₂, pH 7.4 |
| 12 | 50 mM MOPS, 50 mM 2-Glycerol Phosphate disodium Salt, 4.4 mM K2-EDTA,100 mM D-208 | | 51 | 50 mM MOPS, 25 mM 2-Glycerol Phosphate disodium salt, 25 mM D-223, 4.4 mM K2-EDTA, 2 mM Adenine, 25 mM NaOAc, 5 mM KCl, 25 mM Sodium Gluconate, 3 mM MgCl₂, pH 7.5 |
| 13 | 50 mM MOPS, 50 mM 2-Glycerol Phosphate disodium Salt, 4.4 mM K2-EDTA, 100 mM D-209 | | 52 | 50 mM MOPS, 25 mM 2-Glycerol Phosphate disodium salt, 25 mM D-224, 4.4 mM K2-EDTA, 2 mM Adenine, 25 mM NaOAc, 5 mM KCl, 25 mM Sodium Gluconate, 3 mM MgCl₂, pH 7.5 |
| 14 | 50 mM MOPS, 50 mM 2-Glycerol Phosphate disodium Salt, 4.4 mM K2-EDTA, 100 mM D-210 | | 53 | 50 mM MOPS, 25 mM 2-Glycerol Phosphate disodium salt, 50 mM D-225, 4.4 mM K2-EDTA, 2 mM Adenine, 25 mM NaOAc, 5 mM KCl, 25 mM Sodium Gluconate, 3 mM MgCl₂, pH 7.6 |
| 15 | 50 mM MES, 50 mM 2-Glycerol Phosphate disodium Salt, 4.4 mM K2-EDTA, 100 mM D-201 | | 54 | 50 mM MOPS, 25 mM 2-Glycerol Phosphate disodium salt, 50 mM D-226, 4.4 mM K2-EDTA, 2 mM Adenine, 25 mM NaOAc, 5 mM KCl, 25 mM Sodium Gluconate, 3 mM MgCl₂, pH 7.5 |
| 16 | 50 mM MES, 50 mM 2-Glycerol Phosphate disodium Salt, 4.4 mM K2-EDTA, 100 mM D-202 | | 55 | 50 mM MOPS, 25 mM 2-Glycerol Phosphate disodium salt, 50 mM D-227, 4.4 mM K2-EDTA, 2 mM Adenine, 25 mM NaOAc, 5 mM KCl, 25 mM Sodium Gluconate, 3 mM MgCl₂, pH 7.6 |
| 17 | 50 mM MES, 50 mM 2-Glycerol Phosphate disodium Salt, 4.4 mM K2-EDTA, 100 mM D-203 | | 56 | 50 mM MOPS, 25 mM 2-Glycerol Phosphate disodium salt, 50 mM D-228, 4.4 mM K2-EDTA, 2 mM Adenine, 25 mM NaOAc, 5 mM KCl, 25 mM Sodium Gluconate, 3 mM MgCl₂, pH 7.6 |
| 18 | 50 mM MES, 50 mM 2-Glycerol Phosphate disodium Salt, 4.4 mM K2-EDTA, 100 mM D-204 | | 57 | 50 mM MOPS, 25 mM 2-Glycerol Phosphate disodium salt, 50 mM D-229, 4.4 mM K2-EDTA, 2 mM Adenine, 25 mM NaOAc, 5 mM KCl, 25 mM Sodium Gluconate, 3 mM MgCl₂, pH 7.4 |
| 19 | 50 mM MES, 50 mM 2-Glycerol Phosphate disodium Salt, 4.4 mM K2-EDTA, 100 mM D-205 | | 58 | 50 mM MOPS, 25 mM 2-Glycerol Phosphate disodium salt, 50 mM D-230, 4.4 mM K2-EDTA, 2 mM Adenine, 25 mM NaOAc, 5 mM KCl, 25 mM Sodium Gluconate, 3 mM MgCl₂, pH 7.6 |
| 20 | 50 mM MES, 50 mM 2-Glycerol Phosphate disodium Salt, 4.4 mM K2-EDTA, 100 mM D-206 | | 59 | 25 mM MOPSO, 15 mM 2-Glycerol Phosphate disodium salt, 50 mM D-221, 4.4 mM K2-EDTA, 2 mM Adenine, 20 mM NaOAc, 5 mM KCl, 20 mM Sodium Gluconate, 3 mM MgCl₂, pH 7.6 |
| 21 | 50 mM MES, 50 mM 2-Glycerol Phosphate disodium Salt, 4.4 mM K2-EDTA, 100 mM D-207 | | 60 | 25 mM MOPSO, 15 mM 2-Glycerol Phosphate disodium salt, 50 mM D-222, 4.4 mM K2-EDTA, 2 mM Adenine, 20 mM NaOAc, 5 mM KCl, 20 mM Sodium Gluconate, 3 mM MgCl₂, pH 7.4 |
| 22 | 50 mM MES, 50 mM 2-Glycerol Phosphate disodium Salt, 4.4 mM K2-EDTA, 100 mM D-208 | | 61 | 25 mM MOPSO, 15 mM 2-Glycerol Phosphate disodium salt, 50 mM D-223, 4.4 mM K2-EDTA, 2 mM Adenine, 20 mM NaOAc, 5 mM KCl, 20 mM Sodium Gluconate, 3 mM MgCl₂, pH 7.0 |
| 23 | 50 mM MES, 50 mM 2-Glycerol Phosphate disodium Salt, 4.4 mM K2-EDTA, 100 mM D-209 | | 62 | 25 mM MOPSO, 15 mM 2-Glycerol Phosphate disodium salt, 50 mM D-224, 4.4 mM K2-EDTA, 2 mM Adenine, 20 mM NaOAc, 5 mM KCl, 20 mM Sodium Gluconate, 3 mM MgCl₂, pH 7.5 |
| 24 | 50 mM MES, 50 mM 2-Glycerol Phosphate disodium Salt, 4.4 mM K2-EDTA, 100 mM D-210 | | 63 | 25 mM MOPSO, 15 mM 2-Glycerol Phosphate disodium salt, 50 mM D-225, 4.4 mM K2-EDTA, 2 mM Adenine, 20 mM NaOAc, 5 mM KCl, 20 mM Sodium Gluconate, 3 mM MgCl₂, pH 7.2 |
| 25 | 50 mM MES, 50 mM 2-Glycerol Phosphate disodium salt, 100 mM D-211, 4.4 mM K2-EDTA, 2 mM Adenine, pH 6.6 | | 64 | 25 mM MOPSO, 15 mM 2-Glycerol Phosphate disodium salt, 50 mM D-226, 4.4 mM K2-EDTA, 2 mM Adenine, 20 mM NaOAc, 5 mM KCl, 20 mM Sodium Gluconate, 3 mM MgCl₂, pH 7.2 |
| 26 | 50 mM MES, 50 mM 2-Glycerol Phosphate disodium salt, 100 mM D-212, 4.4 mM K2-EDTA, 2 mM Adenine, pH 6.6 | | 65 | 25 mM MOPSO, 15 mM 2-Glycerol Phosphate disodium salt, 50 mM D-227, 4.4 mM K2-EDTA, 2 mM Adenine, 20 mM NaOAc, 5 mM KCl, 20 mM Sodium Gluconate, 3 mM MgCl₂, pH 7.3 |
| 27 | 50 mM MES, 50 mM 2-Glycerol Phosphate disodium salt, 100 mM D-213, 4.4 mM K2-EDTA, 2 mM Adenine, pH 6.6 | | 66 | 25 mM MOPSO, 15 mM 2-Glycerol Phosphate disodium salt, 50 mM D-228, 4.4 mM K2-EDTA, 2 mM Adenine, 20 mM NaOAc, 5 mM KCl, 20 mM Sodium Gluconate, 3 mM MgCl₂, pH 7.0 |
| 28 | 50 mM MES, 50 mM 2-Glycerol Phosphate disodium salt, 100 mM D-214, 4.4 mM K2-EDTA, 2 mM Adenine, pH 6.6 | | 67 | 25 mM MOPSO, 15 mM 2-Glycerol Phosphate disodium salt, 50 mM D-229, 4.4 mM K2-EDTA, 2 mM Adenine, 20 mM NaOAc, 5 mM KCl, 20 mM Sodium Gluconate, 3 mM MgCl₂, pH 7.6 |
| 29 | 50 mM MES, 50 mM 2-Glycerol Phosphate disodium salt, 100 mM D-215, 4.4 mM K2-EDTA, 2 mM Adenine, pH 6.6 | | 68 | 25 mM MOPSO, 15 mM 2-Glycerol Phosphate disodium salt, 50 mM D-230, 4.4 mM K2-EDTA, 2 mM Adenine, 20 mM NaOAc, 5 mM KCl, 20 mM Sodium Gluconate, 3 mM MgCl₂, pH 7.4 |
| 30 | 50 mM MES, 50 mM 2-Glycerol Phosphate disodium salt, 100 mM D-216, 2 mM Adenine, pH 6.6 | | 69 | 25 mM MOPSO, 15 mM 2-Glycerol Phosphate disodium salt, 50 mM myo-inositol, 4.4 mM K2-EDTA, 2 mM Adenine, 20 mM NaOAc, 5 mM KCl, 20 mM Sodium Gluconate, 3 mM MgCl₂, pH 7.6 |
| 31 | 50 mM MES, 50 mM 2-Glycerol Phosphate disodium salt, 100 mM D-217, 4.4 mM K2-EDTA, 2 mM Adenine, pH 6.6 | | 70 | 25 mM MOPSO, 15 mM 2-Glycerol Phosphate disodium salt, 50 mM Gly-Gly-Gly, 4.4 mM K2-EDTA, 2 mM Adenine, 20 mM NaOAc, 5 mM KCl, 20 mM Sodium Gluconate, 3 mM MgCl₂, pH 7.6 |
| 32 | 50 mM MES, 50 mM 2-Glycerol Phosphate disodium salt, 100 mM D-218, 4.4 mM K2-EDTA, 2 mM Adenine, pH 6.6 | | 71 | 25 mM MOPSO, 15 mM 2-Glycerol Phosphate disodium salt, 50 mM Ala-Gln, 4.4 mM K2-EDTA, 2 mM Adenine, 20 mM NaOAc, 5 mM KCl, 20 mM Sodium Gluconate, 3 mM MgCl₂, pH 7.6 |
| 33 | 50 mM MES, 50 mM 2-Glycerol Phosphate disodium salt, 100 mM D-219, 4.4 mM K2-EDTA, 2 mM Adenine, pH 6.6 | | 72 | 25 mM MOPSO, 15 mM 2-Glycerol Phosphate disodium salt, 50 mM N-Methylglucamine, 4.4 mM K2-EDTA, 2 mM Adenine, 20 mM NaOAc, 5 mM KCl, 20 mM Sodium Gluconate, 3 mM MgCl₂, pH 7.6 |
| 34 | 50 mM MES, 50 mM 2-Glycerol Phosphate disodium salt, 100 mM D-220, 4.4 mM K2-EDTA, 2 mM Adenine, pH 6.6 | | 73 | 25 mM MOPSO, 15 mM 2-Glycerol Phosphate disodium salt, 50 mM D-219, 4.4 mM K2-EDTA, 2 mM Adenine, 20 mM NaOAc, 5 mM KCl, 20 mM Sodium Gluconate, 3 mM MgCl₂, pH 7.4 |
| 35 | 50 mM MOPS, 50 mM 2-Glycerol Phosphate disodium salt, 50 mM D-211, 4.4 mM K2-EDTA, 2 mM Adenine, pH 7.4 | | 74 | 50 mM MES, 200 mM Ala-Gln, 50 mM Sucralose, 4.4 mM K2-EDTA, pH 6.9 |
| 36 | 50 mM MOPS, 50 mM 2-Glycerol Phosphate disodium salt, 50 mM D-212, 4.4 mM K2-EDTA, 2 mM Adenine, pH 7.4 | | 75 | 50 mM MOPS, 200 mM Ala-Gln, 25 mM Sucralose, 4.4 mM K2-EDTA, pH 7.2 |
| 37 | 50 mM MOPS, 50 mM 2-Glycerol Phosphate disodium salt, 4.4 mM K2-EDTA, 50 mM D-213, 2 mM Adenine, pH 7.4 | | 76 | 25 mM MES, 25 mM 2-Glycerol Phosphate disodium salt, 100 mM D-219, 4.4 mM K2-EDTA, 5 mM Adenine, 25 mM Sucralose, 25 mM Glucose, 25 mM Ala-Gln, pH 6.9 |
| 38 | 50 mM MOPS, 50 mM 2-Glycerol Phosphate disodium salt, 50 mM D-214, 4.4 mM K2-EDTA, 2 mM Adenine, pH 7.4 | | 77 | 25 mM MES, 25 mM 2-Glycerol Phosphate disodium salt, 50 mM D-219, 4.4 mM K2-EDTA, 5 mM Adenine, 50 mM Sucralose, 25 mM Glucose, 50 mM Ala-Gln, 5 mM KCl, pH 6.9 |
| 39 | 50 mM MOPS, 50 mM 2-Glycerol Phosphate disodium salt, 50 mM D-215, 4.4 mM K2-EDTA, 2 mM Adenine, pH 7.4 | | | |

### METHODS FOR PREPARING FORMULATIONS FOR STABILIZING LEUKOCYTES, ERYTHROCYTES, AND CIRCULATING TUMOR CELLS AT AMBIENT TEMPERATURES

Methods for preparing the formulations described herein for substantially stable storage of a metabolically-active cell in a blood sample at ambient temperatures employ techniques that are well-known to those skilled in the art and generally use commercially available reagents. In some embodiments, the formulations are prepared as concentrated stock solutions of the formulation reagents, e.g., 2X, 5X, 10X, 20X or the like, so as to be admixed with the blood sample at the appropriate ratios to form the desired concentrations. The cationic compounds and zwitterionic compounds disclosed in Table 1 may be synthesized and formulated as previously described, e.g., see WO 20120186638.

### PURIFIED CELLS

In some embodiments, the substantially stable one or more metabolically-active cell are purified from the blood sample using well-known conventional methods routinely employed by those skilled in the art. Apparatus, kits and methods for purifying blood cells from blood are well-known. For instance, a number of principles have been applied to separate erythrocytes from various populations of leukocytes. Traditionally, gradient separations have been used. Gradient separations work on the principle that erythrocytes are small and dense, and can form a pellet when centrifuged, usually below a "cushion" of a substance such as Ficoll. Although effective, the gradient methods are typically slow, difficult to automate, not reproducible, give poor yields, and produce cells with poor viability. Also, the final product often contains remaining erythrocytes contamination that requires additional processing steps (e.g., erythrocytes lysis) to remove the inadequately separated cells.

Another commonly used method to separate blood for functional assays and clinical diagnostics is direct RBC lysis. The principle of this method is that the RBCs are more sensitive to changes in the osmolarity of the media than WBCs, such that a brief and fast change in the osmolarity of the medium or buffer will lyse more RBCs than WBCs. Indeed, lysis methods work, but as with gradient methods, the reproducibility of the separation and the viability and numbers of the remaining WBCs cells are typically poor and not representative of the whole population. Damage to WBCs can also occur with these lysis methods.

A more recently developed technology to separate subpopulations of blood cells has utilized magnetic particles to separate blood cells. Alternatively, substantially stabilized, intact, metabolically active viable cells are purified by affinity chromatography, flow cytometry or by fluorescence activated cell sorting (FACS) analysis using antibodies generated against a native wild type membrane proteins and receptors, which is not possible using storage formulation conditions that denature these cellular proteins. Circulating tumor cells may be isolated using antibodies directed against particular tumor antigens expressed on the cell surface to capture circulating tumor cell populations or specific antibodies may be used to select for specific subpopulations depending on the analysis to be performed and the expression patterns of the circulating tumor cells. The determination of the antigen expression patterns and antibody selection is within the purview of one skilled in the art.

The purified one or more metabolically-active cell may be subsequently stored in the formulations described herein for extended periods before analysis.

### ARTICLES OF MANUFACTURE

In certain embodiments, articles of manufacture are provided in which one of the herein described formulations, including those set forth in Table 2, are contained within a suitable blood collection tube, container or vessel. These articles of manufacture may be used for substantially stable storage of one or more metabolically-active cell by stabilizing the one or more cell at the time of blood collection. In certain embodiments, the blood collection tube is an evacuated blood tube having less than atmospheric pressure to withdraw a predetermined volume of whole blood. In some embodiments, these articles of manufacture are used in the herein described kits and methods.

### KITS

Described herein, in some embodiments, are kits comprising any one of the articles of manufacture comprising the formulations of the present invention, including those in Table 2, and a packaging insert. In some embodiments, the components of the kit are supplied in a container, such as a compartmentalized plastic enclosure. In some embodiments, the container has a hermetically sealable cover so that the contents of the kit can be sterilized and sealed for storage for later use.

### METHODS FOR SUBSTANTIALLY STABLE STORAGE OF ONE OR MORE METABOLICALLY-ACTIVE CELL IN A BLOOD SAMPLE AT AMBIENT TEMPERATURES

In another aspect of the present invention, methods are provided for substantially stable storage of one or more metabolically-active cell in a blood sample at ambient temperatures.

In certain embodiments, the methods comprise admixing a blood sample with formulations described herein for substantially stable storage of metabolically-active cells in a blood sample at ambient temperatures, wherein the cell or at least 80% of the cells remain metabolically-active at room temperature for a period of at least three days. In other embodiments, the cell or cells remain metabolically-active for at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, or at least 18 days.

In certain embodiments, the formulation for substantially stable storage of one or more metabolically-active cell, e.g., a leukocyte, an erythrocyte, and/or a circulating tumor cell, in a blood sample at ambient temperatures comprises: a pH buffer; a chelating agent; and a peptide. In some embodiments, the peptide is a di- or tri-peptide. In some embodiments, the peptide is an alanine-glutamine di-peptide, a glycine-glycine di-peptide or a glycine-glycine-glycine tri-peptide. In certain other embodiments, the formulation for substantially stable storage of one or more metabolically-active cell in a blood sample comprises: a pH buffer; a phosphatase inhibitor; and a purine. In some embodiments, the purine is adenine or guanine. In some embodiments, the formulations further comprise a chelating agent and at least one of a cationic compound, zwitterionic compound or a peptide, and may still further comprise an acetate buffer, sucralose, glucose, potassium chloride, myo-inositol, N-methylglucamine, magnesium chloride, or a combination thereof. In some embodiments, the formulations further comprise a chelating agent and at least one of a cationic compound, zwitterionic compound or a peptide, and still further comprise an acetate buffer, sucralose, glucose, potassium chloride, myo-inositol, N-methylglucamine, magnesium chloride, or a combination thereof. In certain embodiments, the formulation is one of the formulations set forth in Table 2.

Blood collection tubes, bags, containers and vessels are well-known in the art and have been employed by medical practitioners for decades. Blood collected for substantially stable storage of one or more metabolically-active cell may be obtained using any method or apparatus commonly employed by those skilled in the art such as venipuncture or finger prick. In some embodiments, when the blood is collected by venipuncture, the formulation is located inside the blood collection tube, e.g., an evacuated tube (VACUTAINER blood collection tube, Becton Dickenson or VACUETTE blood collection tube, Greiner Bio-One) at the time that the blood sample is obtained from the subject. In some embodiments, when the blood is collected by venipuncture, the formulations is added to an already obtained whole blood sample, either immediately or shortly after it is withdrawn.

The methods as described herein may use the articles of manufacture and kits disclosed herein.

The following Examples are presented by way of illustration and not limitation.

### EXAMPLE 1 STABILIZATION OF INTACT, METABOLICALLY-ACTIVE LEUOKOCYTES AND ERYTHROCYTES IN HUMAN BLOOD SAMPLE FOR AT LEAST 18 DAYS AT AMBIENT TEMPERATURES

This Example describes formulations and compositions of the present invention for stabilizing leukocytes and/or erythrocytes in a blood sample for a period of at least 18 days at ambient temperatures.

Whole human blood was collected from a consenting donor in K2-EDTA tubes from Greiner Bio-One (Cat# 455045). 1.5 mL of each formulation was placed into 3 mL empty Vacuette tubes from Greiner Bio-One. The blood was pooled into a single container, mixed and distributed to each of the tubes, with mixing after every 10 aliquots of blood to tubes. The tubes were capped and mixed 10-12 times by inversion. Tubes were stored at ambient temperature in a lab at 75°F (23.9 °C). Duplicate tubes were prepared for each formulation and two tubes were additionally prepared for both the room temperature control and 4°C control, respectively. The controls were prepared with 3 mL of blood each in order to cleanly obtain the plasma from sample aliquots. After 4 days the untreated blood was lysed by freezing and thawing the blood twice then diluting the blood 1:1 to account for the addition of the stabilizer to each sample. The blood was then serially diluted 10 fold to give a 10% hemolysis control and then diluted another 5 fold to give a 2% hemolysis control. The 2% hemolysis control was diluted sequentially 1:1 to give additional dilutions of 1, 0.5, 0.25, 0.125, 0.0625, 0.03125, and 0.015625. 200 microliter aliquots of the dilutions were transferred to a 96-well UV-star microtiter plate (Greiner Bio-One) and measurements taken at 415 nm using a Biotek Synergy 2 instrument according to the manufacturer's instructions. The percent hemolysis values were plotted against the OD at 415 nm and used to calculate the regression line through the points. The equation that defines the calculated regression line was obtained through the use of excel and this line was used to calculate the percent hemolysis for a subset of formulations analyzed from Table 2 being tested at 4, 10 and 18 days.

**TABLE 3. Determination Regression Line of Percent Hemolysis for Control Samples**

| % Hemolysis | #1 | #2 | Average | % Hemolysis |
|---|---|---|---|---|
| 1 | 6.177 | 6.324 | 6.2505 | 1 |
| 0.5 | 3.118 | 3.234 | 3.176 | 0.5 |
| 0.25 | 1.637 | 1.632 | 1.6345 | 0.25 |
| 0.125 | 0.816 | 0.829 | 0.8225 | 0.125 |
| 0.0625 | 0.404 | 0.415 | 0.4095 | 0.0625 |
| 0.03125 | 0.209 | 0.211 | 0.21 | 0.03125 |
| 0.015625 | 0.11 | 0.119 | 0.1145 | 0.015625 |

The resulting regression line was calculated from these data to be y = 0.1603x - 0.0055 and an R2 value equal to 0.9999.

Sample preparation at each condition and time point included removal of 1 mL aliquots from each of the tube and transfer to a 1.5 mL Eppendorf microfuge tube. The tubes were spun at 3000 rpm for 5 min in an Eppendorf 5417 centrifuge and 250 microliter aliquots of the plasma layer were transferred from each tube and diluted 1:1 at the 4 day time points. Later time points for some formulations including the controls required greater dilutions so that the reading would not be off scale. All measurements were corrected by water blanks and corrected for the pathlength via the Gen 5 software used to control the Biotek plate reader in accordance with the manufacturer's instructions. The percent hemolysis of the two reading duplicates was averaged. Table 4 lists the values for each duplicate sample (i.e., "#1" and "#2"), an average value, and percent hemolysis observed on Days 4, 10 and 18 for each formulation tested (formulation numbers 1-24 from Table 2).

**TABLE 4. Stabilization of Cells in Blood Samples Stored at Ambient Temperatures for Four, Ten or Eighteen Days Using Exemplary Formulations of Table 2**

| Form | 4 Day #1 | 4 Day #2 | Avg | % Hemolysis | 10-Day #1 | 10 Day #2 | Avg | % Hemolysis | 18 Day #1 | 18 Day #2 | Avg | %Hemolysis |
|---|---|---|---|---|---|---|---|---|---|---|---|---|
| NP @ RT | 1.008 | 1.102 | 1.055 | 0.327 | 6.512 | 6.521 | 6.516 | 4.156 | 5.503 | 5.581 | 5.542 | 14.126 |
| NP @ 4 °C | 1.201 | 1.189 | 1.195 | 0.372 | 1.350 | 1.345 | 1.348 | 0.842 | 5.849 | 5.825 | 5.837 | 7.441 |
| 1 | 0.712 | 0.718 | 0.715 | 0.218 | 2.911 | 2.868 | 2.890 | 0.915 | 2.910 | 2.899 | 2.904 | 1.840 |
| 2 | 0.801 | 0.797 | 0.799 | 0.245 | 3.713 | 3.707 | 3.710 | 1.178 | 6.355 | 6.229 | 6.292 | 4.012 |
| 3 | 0.749 | 0.755 | 0.752 | 0.230 | 2.656 | 2.682 | 2.669 | 0.845 | 1.724 | 1.710 | 1.717 | 1.079 |
| 4 | 0.827 | 0.819 | 0.823 | 0.253 | 3.169 | 3.174 | 3.172 | 1.006 | 1.496 | 1.505 | 1.500 | 0.940 |
| 5 | 1.010 | 1.003 | 1.007 | 0.312 | 3.248 | 3.241 | 3.244 | 1.029 | 4.031 | 4.077 | 4.054 | 2.577 |
| 6 | 0.963 | 0.926 | 0.944 | 0.292 | 4.165 | 4.153 | 4.159 | 1.322 | 5.567 | 5.479 | 5.523 | 3.519 |
| 7 | 0.810 | 0.811 | 0.810 | 0.249 | 3.139 | 3.116 | 3.128 | 0.992 | 3.889 | 3.957 | 3.923 | 2.493 |
| 8 | 0.800 | 0.787 | 0.794 | 0.243 | 4.336 | 4.252 | 4.294 | 1.366 | 4.494 | 4.489 | 4.492 | 2.858 |
| 9 | 0.772 | 0.744 | 0.758 | 0.232 | 2.889 | 2.835 | 2.862 | 0.907 | 3.823 | 3.820 | 3.822 | 2.428 |
| 10 | 1.009 | 1.008 | 1.008 | 0.312 | 2.893 | 2.846 | 2.870 | 0.909 | 3.368 | 3.363 | 3.366 | 2.136 |
| 11 | 1.248 | 1.260 | 1.254 | 0.391 | 3.710 | 3.738 | 3.724 | 1.183 | 5.267 | 5.331 | 5.299 | 3.376 |
| 12 | 1.083 | 1.080 | 1.082 | 0.336 | 3.864 | 3.875 | 3.870 | 1.230 | 4.994 | 4.966 | 4.980 | 3.171 |
| 13 | 0.778 | 0.787 | 0.782 | 0.240 | 3.037 | 3.022 | 3.030 | 0.960 | 3.526 | 3.549 | 3.538 | 2.246 |
| 14 | 1.021 | 1.001 | 1.011 | 0.313 | 5.122 | 5.090 | 5.106 | 1.626 | Overflow | Overflow | | |
| 15 | 0.710 | 0.709 | 0.710 | 0.216 | 2.665 | 2.627 | 2.646 | 0.837 | 3.399 | 3.316 | 3.358 | 2.131 |
| 16 | 0.799 | 0.808 | 0.804 | 0.247 | 3.896 | 3.953 | 3.924 | 1.247 | 4.445 | 4.439 | 4.442 | 2.826 |
| 17 | 1.022 | 1.030 | 1.026 | 0.318 | 3.107 | 3.055 | 3.081 | 0.977 | 2.856 | 2.895 | 2.876 | 1.822 |
| 18 | 1.049 | 1.040 | 1.044 | 0.324 | 3.601 | 3.633 | 3.617 | 1.149 | 4.729 | 4.724 | 4.726 | 3.009 |
| 19 | 1.013 | 1.026 | 1.020 | 0.316 | 2.749 | 2.795 | 2.772 | 0.878 | 3.805 | 3.786 | 3.796 | 2.412 |
| 20 | 0.886 | 0.869 | 0.878 | 0.270 | 1.413 | 1.412 | 1.412 | 0.442 | 2.936 | 2.959 | 2.948 | 1.868 |
| 21 | 1.032 | 1.038 | 1.035 | 0.321 | 1.590 | 1.589 | 1.590 | 0.499 | 3.092 | 3.069 | 3.080 | 1.953 |
| 22 | 1.002 | 0.972 | 0.987 | 0.306 | 2.298 | 2.309 | 2.304 | 0.728 | 6.723 | 6.350 | 6.536 | 4.169 |
| 23 | 0.714 | 0.713 | 0.714 | 0.218 | 1.196 | 1.170 | 1.183 | 0.368 | 4.282 | 4.280 | 4.281 | 2.723 |
| 24 | 0.820 | 0.796 | 0.808 | 0.248 | 1.889 | 1.877 | 1.883 | 0.593 | 4.014 | 4.014 | 4.014 | 2.552 |

As shown in Table 4, the subset of the exemplary formulations tested for stabilizing leukocytes and erythrocytes prevented hemolysis of erythrocytes as well or better than samples stored unprotected at 4°C for the same time period of at least 18 days.

### EXAMPLE 2 STABILIZATION OF NATIVE CELL SURFACE PROTEINS EXPRESSED ON LEUKOCYTES IN A HUMAN BLOOD SAMPLE FOR AT LEAST SIX DAYS AT AMBIENT TEMPERATURES

This example demonstrated that formulations and compositions of the present invention stabilized leukocytes, lymphocytes and granulocytes in a blood sample at ambient temperature for a period of up to 6 days, as evidenced by FACS analysis using antibodies targeting native, exemplary protein markers expressed on the surface of various leukocytes, CD45, CD4, CD8, and CD66b.

### A. CD45-positive leukocytes

The leukocyte specific marker, CD45, was used to evaluate the relative stability of the leukocyte population in whole blood compositions comprising formulations of the present invention.

Whole human blood collected in K2-EDTA VACUETTE tubes was admixed at a 1:1 ratio of whole blood with 1X stocks of the formulations 33, 74, 75, 76, or 77 of Table 2. Control samples were stored at room temperature (RT) and 4°C. At 0, 3 and 6 days post RT storage the experimental and control samples were retrieved from storage and labeled using a CD45 FITC-conjugated antibody according the manufacturer's instructions. Erythrocytes were removed by treating the blood samples with BD FACS lysing solution (Cat# 216667-08-2) or a generic non-fixative lysis buffer for 10 minutes prior to 2 washes using 1X phosphate buffered saline (PBS). The remaining cells, predominantly leukocytes, were suspended in 1X PBS and analyzed on the BD ACCURI Flow Cytometer at 100,000 events/sample and a medium flow rate setting. COUNTBRIGHT counting beads (Life Techologies Cat# C36950) were added per the manufacturer's instructions to obtain an accurate cell count. BD VIA-PROBE (Cat# 555816) was added per the manufacturer's instructions to assess viability. Data were acquired and analyzed to evaluate side scatter versus CD45-FITC/FL-1 fluorescence.

Fig. 1A shows the flow cytometry data from the control samples at 0 and 6 days post room temperature storage. In the control samples the granulocyte population separated into two distinct populations, one with higher and one with lower CD45 fluorescence. In the unprotected controls the granularity in the granulocyte population changed, which was indicated by the reduction in the SSC. There was little change in granularity and no separation of granulocyte populations in formulations 74 and 76 at day 6 post collection. The granulocyte populations in formulation 74 and 76 required only a slight gate adjustment between days 0 and day 6, while the unprotected controls required large gating changes. Additionally, there was no defined monocyte population to gate in the unprotected samples at day 6, while these populations were distinct at day 0 and in formulations 74 and 76 at day 6.

Fig. 1B shows the flow cytometry data from the control samples at 0 and 6 days post 4°C storage. Similar to the room temperature studies, by day 6 the monocyte population in the unprotected samples shifted, making gating unreliable. Formulations 74 and 75 maintained a monocyte population with little shift in gating.

Formulations 33, 74, 75 and 77 resulted in high recovery (>60%) of granulocytes, lymphocytes and monocytes at 4°C on day 6 compared to day 0 values (Fig. 2A). Many (30-87%) of these granulocytes, lymphocytes and monocytes were alive at day 6 (Fig. 2B).

Formulations 33, 74, 75 and 77 resulted in good recovery (45->100%) of granulocytes, lymphocytes and monocytes at room temperature on day 6 compared to day 0 values (Fig. 3A). Many (>20%) of these granulocytes, lymphocytes and monocytes were alive at day 6 (Fig. 3B).

### B. CD45-positive monocytes

Whole human blood collected in K2-EDTA tubes was mixed with an equal volume (1 mL) of a 1X concentration of formulation 76 of Table 2. The samples were stored at room temperature for 0 or 6 days prior to analysis. Untreated control samples were stored at 4°C and processed in parallel with test samples. An anti-CD45-FITC antibody was added per the manufacturer's instructions. The erythrocytes were lysed with BD FACS lysing solution (Cat# 216667-08-2), washed twice with PBS and resuspended in 200 µL of 1X PBS. The remaining cells, predominantly leukocytes, were suspended in PBS and analyzed on the BD ACCURI flow cytometer at 100,000 events/sample, all data were acquired and the analysis was limited to side scatter vs CD45-FITC/FL-1 fluorescence.

As shown in Figure 4, formulation 76 was capable of stabilizing the monocyte population at 4°C whereas the monocyte population could not be accurately gated in the non-protected control.

### C. CD4- and CD8-positive T cells/lymphocytes

Whole human blood collected in K2-EDTA tubes was mixed a 1X concentration of formulations 33 and 77 of Table 2. The samples were stored at room temperature for 0 or 5 days prior to analysis. Untreated control samples were stored at 4°C and processed in parallel with test samples. An anti-CD4-FITC and CD8-APC antibody was added per the manufacturer's recommendations. The erythrocytes were lysed with BD FACS lysing solution (Cat# 216667-08-2), washed twice with PBS and resuspended in 200 µL of 1X PBS. The remaining cells were suspended in 1X PBS and analyzed on the BD ACCURI flow cytometer at 100,000 events/sample. The analysis was limited to side scatter vs CD45-FITC/FL-1 fluorescence.

As shown in Fig. 5, the number of CD4- and CD8-positive lymphocytes decreased to approximately 20% of the day 0 values by day 5 at 4°C (unprotected sample). Over twice as many CD4- and CD8-positive lymphocytes were present at day 5 in formulations 33 and 77.

### D. CD66b-positive granulocytes

Whole human blood collected in K2-EDTA tubes was mixed with a 1X concentration of formulations 33 or 77 of Table 2. The samples were stored at room temperature for 6 hours prior to analysis. Untreated control samples were stored at 4°C and processed in parallel with test samples. An anti-CD66b-FITC antibody and BD VIAPROBE was added per the manufacturer's recommendations to label the granulocytes and assess cell viability respectively. The erythrocytes were lysed with BD FACS lysing solution (Cat# 216667-08-2), washed twice with PBS and resuspended in 200 µL of 1X PBS. The remaining cells were suspended in 1X PBS and analyzed on the BD ACCURI flow cytometer at 100,000 events/sample. The analysis was limited to gating on CD66b-FITC/FL-1 fluorescence and then evaluating the FSC vs viability (FL3 fluorescence).

As shown in Fig. 6, formulation 33 was capable of stabilizing intact granulocytes in a blood sample for a period of 8 hours at ambient temperatures compared to an unprotected control. In this experiment 70% of the granulocytes were viable after 8 hours in an unprotected sample, while 98.8% of the granulocytes stabilized by formulation 33 were viable after 8 hours. These results demonstrated the ability of the formulations and compositions of the present invention to stabilize viable intact CD66b-positive granulocytes.

Unless the context requires otherwise, throughout the present specification and claims, the word "comprise" and variations thereof, such as, "comprises" and "comprising," which is used interchangeably with "including," "containing," or "characterized by," is inclusive or open-ended language and does not exclude additional, unrecited elements or method steps. The phrase "consisting of' excludes any element, step, or ingredient not specified in the claim. The phrase "consisting essentially of' limits the scope of a claim to the specified materials or steps and those that do not materially affect the basic and novel characteristics of the claimed invention. The present disclosure contemplates embodiments of the invention compositions and methods corresponding to the scope of each of these phrases. Thus, a composition or method comprising recited elements or steps contemplates particular embodiments in which the composition or method consists essentially of or consists of those elements or steps.

Reference throughout this specification to "one embodiment" or "an embodiment" or "an aspect" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The various embodiments described above can be combined to provide further embodiments. These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

In summary, the following items are disclosed herewith:
1. A formulation for substantially stable storage of one or more metabolically-active cell in a blood sample at ambient temperatures, wherein the one or more cell remains metabolically-active after storage at room temperature for a period of at least three days.
2. The formulation of item 1, wherein at least 80% of the substantially stored cells remain metabolically-active after storage at room temperature for a period of at least three days.
3. The formulation of item 1 or item 2, wherein at least 80% of the cells remain metabolically-active at room temperature for a period of at least 18 days.
4. The formulation of any one of items 1-3, comprising:
   (i) a pH buffer;
   (ii) a chelating agent; and
   (iii) a peptide.
5. The formulation of item 4, wherein the pH buffer is selected from the group consisting of 2-(N-morpholino)ethanesulfonic acid (MES), 3-(N-morpholino)propanesulfonic acid (MOPS), 3-morpholino-2-hydroxypropanesulfonic acid (MOPSO), and a combination thereof.
6. The formulation of any one of items 4-5, wherein the peptide is a di-peptide or a tri-peptide.
7. The formulation of any one of items 4-6, wherein the di-peptide sequence is Ala-Gln or Gly-Gly and the tri-peptide sequence is Gly-Gly-Gly.
8. The formulation of any one of items 4-7, wherein the chelating agent is EDTA.
9. The formulation of any one of items 1-8, wherein the one or more metabolically-active cell is selected from the group consisting of a leukocyte, an erythrocyte, a circulating tumor cell, and a combination thereof.
10. The formulation of any one of items 1-3, comprising:
   (i) a pH buffer;
   (ii) a chelating agent;
   (iii) a phosphatase inhibitor; and
   (iv) a purine.
11. The formulation of item 10, wherein the phosphatase inhibitor is a serine-threonine phosphatase inhibitor.
12. The formulation of item 11, wherein the serine-threonine phosphatase inhibitor is 2-glycerol phosphate.
13. The formulation of item 10, wherein the purine is adenine or guanine.
14. The formulation of item 13, wherein the purine is adenine.
15. The formulation of item 13, wherein the purine is guanine.
16. The formulation of any one of items 10-15, further comprising a chelating agent, and at least one compound selected from the group consisting of a cationic compound, a zwitterionic compound, and a peptide.
17. The formulation of item 16, wherein the chelating agent is sodium gluconate.
18. The formulation of any one of items 16-17, wherein the zwitterionic compound is a compound of formula (I): 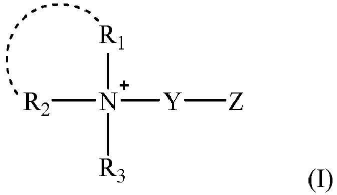 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), wherein A is an unsubstituted or substituted alkyl, aryl, arylalkyl, or any side chain typically found in one of the 20 naturally occurring amino acids; and Z is CO₂-, SO₃- or OPO₃-.
19. The formulation of any one of items 16-17, wherein the cationic compound is selected from the group consisting of:
   (a) a compound of formula (II): 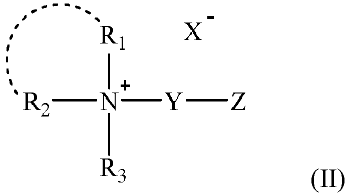 wherein R1, R2, and R3 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; Z is CO₂A; and X is a pharmaceutically acceptable anion;
   (b) a compound of formula (III): 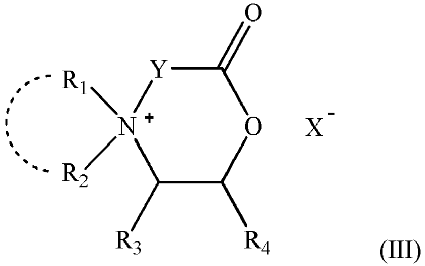 wherein R1, R2, R3, and R4 are independently selected from unsubstituted or substituted alkyl, unsubstituted or substituted aryl, unsubstituted or substituted arylalkyl, or R1 and R2 optionally form a ring, Y is CH₂, CH(A), CH(A)-CH(A), CH(A)-CH(A)-CH(A), where A is an unsubstituted or substituted alkyl, aryl, arylalkyl or any side chain typically found in one of the 20 naturally occurring amino acids; and X is a pharmaceutically acceptable anion; and
   (c) a compound of formula (IV): 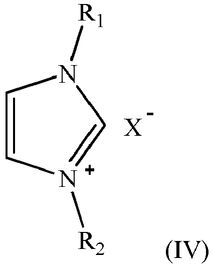 wherein R1 and R2 are independently selected from unsubstituted or substituted alkyl, unsubstituted or substituted aryl, unsubstituted or substituted arylalkyl; and X is a pharmaceutically acceptable anion.
20. The formulation of item 18 or item 19, wherein R1 and R2 of a compound of formula (I), formula (II), or formula (III) form a morpholino ring, pyrrolidinium ring, a piperidinium ring, or an oxazinium ring.
21. The formulation of item 16, wherein the zwitterionic compound is selected from the zwitterionic compounds set forth in Table 1.
22. The formulation of item 16, wherein the cationic compound is selected from the cationic compounds set forth in Table 1.
23. The formulation of item 16, wherein the zwitterionic compound is a quaternary inner salt.
24. The formulation of item 23, wherein the quaternary inner salt is selected from the group consisting N,N-dimethyl-N-(2-hydroxyethyl)-3-ammonium-proprionate or N-ethyl-piperidinium-4-butylsulfonate.
25. The formulation of any one of items 16-24, wherein the peptide has the amino acid sequence of Ala-Gln, Gly-Gly, or Gly-Gly-Gly.
26. The formulation of any one of items 10-25, further comprising an acetate buffer, sucralose, glucose, potassium chloride, myo-inositol, N-methylglucamine, magnesium chloride, or a combination thereof.
27. The formulation of any one of items 10-26, wherein the one or more metabolically-active cell is selected from the group consisting of a leukocyte, an erythrocyte, a circulating tumor cell, and a combination thereof.
28. The formulation of any one of items 1-3, wherein the formulation is selected from the formulations set forth in Table 2.
29. A composition comprising a substantially, stably stored one or more purified, metabolically-active leukocyte admixed with the formulation any one of items 1-8 and 10-28.
30. A composition comprising a substantially, stably stored one or more purified, metabolically-active erythrocyte admixed with the formulation any one of items 1-8 and 10-28.
31. A composition comprising a substantially, stably stored one or more purified, metabolically-active circulating tumor cell admixed with the formulation any one of items 1-8 and 10-28.
32. An article of manufacture, comprising the formulation of any one of items 1-28 contained within a blood collection tube.
33. The article of manufacture of item 32, wherein the blood collection tube is an evacuated blood collection tube.
34. A kit, comprising any one of the articles of manufacture of item 32 or 33 and a package insert.
35. A method for substantially stable storage of one or more metabolically-active cell in a blood sample at ambient temperatures, comprising: admixing a sample of collected blood from a subject with the formulations any one of items 1-28, wherein the one or more cell remain metabolically-active at room temperature for a period of at least three days.
36. The method of item 35, wherein the cell remains metabolically-active at room temperature for a period of at least 18 days.
37. The method of item 35 or item 36, wherein the cell is a leukocyte, an erythrocyte a circulating tumor cell, or a combination thereof.
38. The method of any one of items 35-37, wherein the subject is an animal.
39. The method of any one of items 35-37, wherein the subject is a mammal.
40. The method of any one of items 35-37, wherein the subject is a human.

## Claims

1. A blood collection tube for stable storage of cells in a blood sample at ambient temperatures comprising:
(i) a pH buffer;
(ii) a chelating agent;
(iii) optionally sucralose; and
(iv) at least one compound selected from the group consisting of a peptide, a cationic compound, and a zwitterionic compound.

2. The blood collection tube of claim 1, wherein the pH buffer is selected from the group consisting of citric acid, tartaric acid, malic acid, sulfosalicylic acid, sulfoisophthalic acid, oxalic acid, borate, CAPS (3-(cyclohexylamino)-1-propanesulfonic acid), CAPSO (3-(cyclohexylamino)-2-hydroxy-l-propanesulfonic acid), EPPS (4-(2-hydroxyethyl)-1-piperazinepropanesulfonic acid), HEPES (4-(2-hydroxyethyl)piperazine-1-ethanesulfonic acid), MES (2-(N-morpholino)ethanesulfonic acid), MOPS (3-(N-morpholino)propanesulfonic acid), MOPSO (3-morpholino-2-hydroxypropanesulfonic acid), PIPES (1,4-piperazinediethanesulfonic acid), TAPS (N-[tris(hydroxymethyl)methyl]-3-aminopropanesulfonic acid), TAPSO (2-hydroxy-3-[tris(hydroxymethyl)methylamino]-1-propanesulfonic acid), TES (N-[tris(hydroxymethyl)methyl]-2-aminoethanesulfonic acid), bicine (N,N-bis(2-hydroxyethyl)glycine), tricine (N-[tris(hydroxymethyl)methyl]glycine), tris(tris(hydroxymethyl)aminomethane) and bis-tris (2-[bis(2-hydroxyethyl)amino]-2-(hydroxymethyl)-1,3-propanediol), and any combinations thereof.

3. The blood collection tube of claim 1 or claim 2, wherein the chelating agent comprises EDTA, sodium gluconate, or a combination thereof.

4. The blood collection tube of any of claims 1 to 3, wherein the peptide is a di-peptide or a tri-peptide.

5. The blood collection tube of claim 4, wherein the di-peptide or the tri-peptide comprises alanine (Ala), glutamine (Gln), and/or glycine (Gly).

6. The blood collection tube of claim 5, wherein the di-peptide is Ala-Gln or Gly-Gly, and the tri-peptide is Gly-Gly-Gly.

7. The blood collection tube of any of claims 1 to 6, wherein the cationic compound is selected from the group consisting of 4-(2-ethoxy-2-oxoethyl)-4-ethylmorpholin-4-ium bromide, N-(2-ethoxy-2-oxoethyl)-3-hydroxy-N,N-bis(2-hydroxyethyl)propan-1-aminium bromide, 2-ethoxy-N,N,N-triethyl-2-oxoethanaminium bromide, 1-(2-ethoxy-2-oxoethyl)-4-hydroxy-1-methylpiperidinium bromide, 2-ethoxy-N-(2-(2-hydroxyethoxy)ethyl)-N,N-dimethyl-2-oxoethanaminium bromide, 4-(2-hydroxyethyl)-4-methyl-2-oxomorpholin-4-ium bromide, 4-(2-ethoxy-2-oxoethyl)-4-methylmorpholin-4-ium bromide, 1-(2-ethoxy-2-oxoethyl)-1-methylpyrrolidinium bromide, 3-(2,3-dihydroxypropyl)-1-methyl-1H-imidazol-3-ium chloride, 1,3-dimethyl-1H-imidazol-3-ium methyl sulfate, 3-(2-ethoxy-2-oxoethyl)-1-methyl-1H-imidazol-3-ium bromide, N-benzyl-2-ethoxy-N,Ndimethyl-2-oxoethanaminium bromide, 2-ethoxy-N,N-diethyl-N-methyl-2-oxoethanaminium bromide, N-(2-ethoxy-2-oxoethyl)-N,N-dimethylbutan-1 -aminium bromide, 1-(2-ethoxy-2-oxoethyl)-1-methylpiperidinium bromide, N-(2-ethoxy-2-oxoethyl)-N,N-dimethylbenzenaminium bromide, 1-(2-ethoxy-2-oxoethyl)-3-hydroxy-1-methylpiperidinium bromide, 3-(2-(2-hydroxyethoxy)ethyl)-1-methyl-1H-imidazol-3-ium chloride, 3-(2-(2-(2-hydroxyethoxy)ethoxy)ethyl)-1-methyl-1H-imidazol-3-ium chloride, 1-methyl-3-tetradecyl-1Himidazol-3-ium bromide, and N-(2-ethoxy-2-oxoethyl)-N,N-dimethylcyclohexanaminium bromide.

8. The blood collection tube of any of claims 1 to 7, wherein the zwitterionic compound is selected from the group consisting of 2-(benzyl(2-hydroxyethyl)(methyl)ammonio)acetate, a quaternary inner salt, N,N-dimethyl-N-(2-hydroxyethyl)-3-ammonium-proprionate, N-ethyl-piperidinium-4-butylsulfonate, 2-((3-hydroxypropyl)dimethylammonio)acetate, 2-((2-hydroxypropyl)dimethylammonio)acetate, 2-(2-(hydroxymethyl)-1-methylpiperidinium-1-yl)acetate, 2-((2-hydroxyethyl)dimethylammonio)acetate, 2-((2,3-dihydroxypropyl) dimethylammonio)acetate, 2-(4-hydroxy-1-methylpiperidinium-1-yl)acetate, 2-((2-(2-hydroxyethoxy)ethyl)dimethylammonio)acetate, 2-(bis(2-hydroxyethyl)-(methyl)ammonio)acetate, 2-(4-(2-hydroxyethyl)morpholino-4-ium)acetate, 2-(4-(2-hydroxyethyl)morpholino-4-ium)acetate, 2-(1-(2-hydroxyethyl) pyrrolidinium-1-yl) acetate, and 3-((2-hydroxy-ethyl)dimethylammonio) propanoate.

9. The blood collection tube of any of claims 1 to 8, further comprising an acetate buffer, glucose, potassium chloride, myo-inositol, N-methylglucamine, magnesium chloride, a phosphatase inhibitor, or any combination thereof.

10. The blood collection tube of any of claims 1 to 9, wherein the cells comprise one or more of leukocytes, erythrocytes, tumor cells, or any combination thereof.

11. The blood collection tube of any of claims 1 to 10, wherein the cells are stable at ambient temperature for at least three days.

12. A method for stable storage of cells in a blood sample at ambient temperature, the method comprising:
collecting a blood sample from a subject using any of the blood collection tubes of claims 1 to 11, wherein the cells are stable at ambient temperature for at least three days.

13. The method of claim 12, wherein the blood collection tube is an evacuated blood collection tube.

14. The method of claim 12 or claim 13, wherein the cells comprise one or more of leukocytes, erythrocytes, tumor cells, or any combination thereof.

15. The method of any of claims 12 to 14, wherein at least one of the cells are metabolically active.
